# EUROPEAN PATENT APPLICATION

(11) **EP 2 784 743 A1**
(43) Date of publication of application: **01.10.2014**
(21) Application number: 14160853.9
(22) Date of filing: 20.03.2014
(51) Int. Cl.: G06Q 50/24, G06Q 10/10

(54) **Conference support system, conference support method, and program**

(30) Priority: 29.03.2013 JP 2013073017
(71) Applicant: CANON KABUSHIKI KAISHA, Ohta-ku Tokyo 146-8501 (JP)
(72) Inventor: Kurosawa, Takahiro, Ohta-ku, Tokyo (JP)
(74) Representative: Derham, Cassandra Virginie

(57) **Abstract**

A medical conference support system (1,2) includes a selection means configured to select a conference from a plurality of conferences, and an obtaining means configured to obtain medical information using a search condition determined based on a conference selected by the selection means and information indicating a search condition associated with each of the plurality of conferences.

## Description

### BACKGROUND OF THE INVENTION

### Field of the Invention

The present invention relates to a conference support system, a conference support method, and a storage medium storing a program therefor.

### Description of the Related Art

Recently, there has been an increase in conferences (i.e., medical conferences) in the medical field. Such conferences are held for determining or transmitting diagnosis and observations and treatment plans to nursing and caring teams. Since current medical practice is shifting from a single approach by an individual physician to a team approach by a plurality of physicians and staff members, such conferences are increasing. On the other hand, a large amount of time is still necessary for performing examinations and treatments in a medical site, so that it is difficult to allocate time to preparations for and holding the medical conference.

Further, many hospitals currently use a Picture Archiving and Communication System (PACS) conforming to a Digital Imaging and Communication in Medicine (DICOM) standard.

On the other hand, the standard for electronic medical records such as Health Level Seven Clinical Document Architecture (HL7 CDA) has been proposed. However, HL7 CDA has not yet been widely used.

Furthermore, an examination report (hereinafter referred to as a report) management system manages the reports written by a radiologist or a pathologist. Moreover, in recent years, a large number of image data (i.e., non-DICOM images) other than the DICOM images managed by PACS have been used in the medical sites. A typical example is a photographic image of an external wound or an affected area before and after an operation, which is captured using a digital camera. Such non-DICOM images are often managed by an image management system other than the PACS.

Discussions are carried on in the above-described medical conference by referring to information stored in the electronic medical records, PACS, the report management system, and/or the image management system via respective applications thereof.

Japanese Patent Application Laid-Open No. 2009-193157 discusses a method for collecting materials to be referred to in the medical conference. More specifically, the materials which are related to a diagnosis name and a current status of the treatment are selected from a group of materials obtained from a plurality of medical examination systems.

### SUMMARY OF THE INVENTION

However, according to the method discussed in Japanese Patent Application Laid-Open No. 2009-193157, it is necessary for the physician to input the diagnosis name and the current status of the treatment for collecting the materials to be referred to in the medical conference. It is thus burdensome for the physician.

The present invention is directed to a technique for reducing the time for collecting the materials to be referred to in the medical conference.

The present invention is not limited to the above-described objective. The present invention is also directed to a technique for achieving an effect lead from each of configurations illustrated in exemplary embodiments of the present invention to be described below which cannot be obtained by conventional techniques.

According to a first aspect of the present invention, there is provided a medical conference support system as specified in claims 1 to 6. According to a second aspect of the present invention, there is provided a method for controlling a medical conference support system as specified in clams 7 to 12. According to a third aspect of the present invention, there is provided a computer executable program for causing a computer to execute the conference support method as specified in clams 13.

Further features of the present invention will become apparent from the following description of exemplary embodiments with reference to the attached drawings.

### BRIEF DESCRIPTION OF THE DRAWINGS

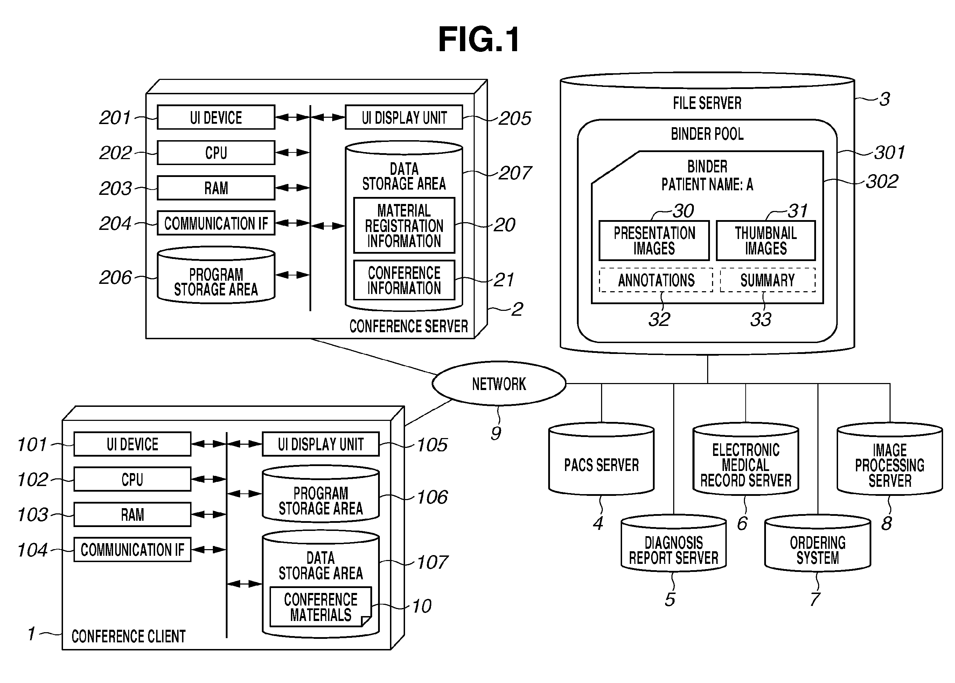

Fig. 1 illustrates a typical usage example employing the present invention.

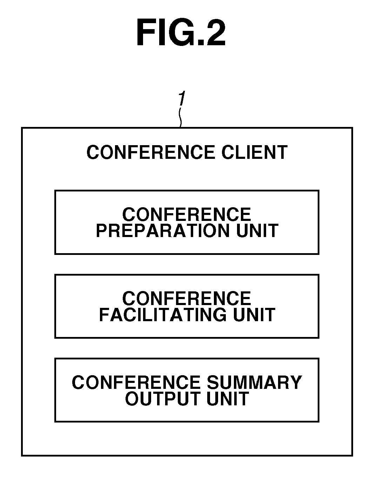

Fig. 2 is a schematic diagram illustrating an example of a program configuration.

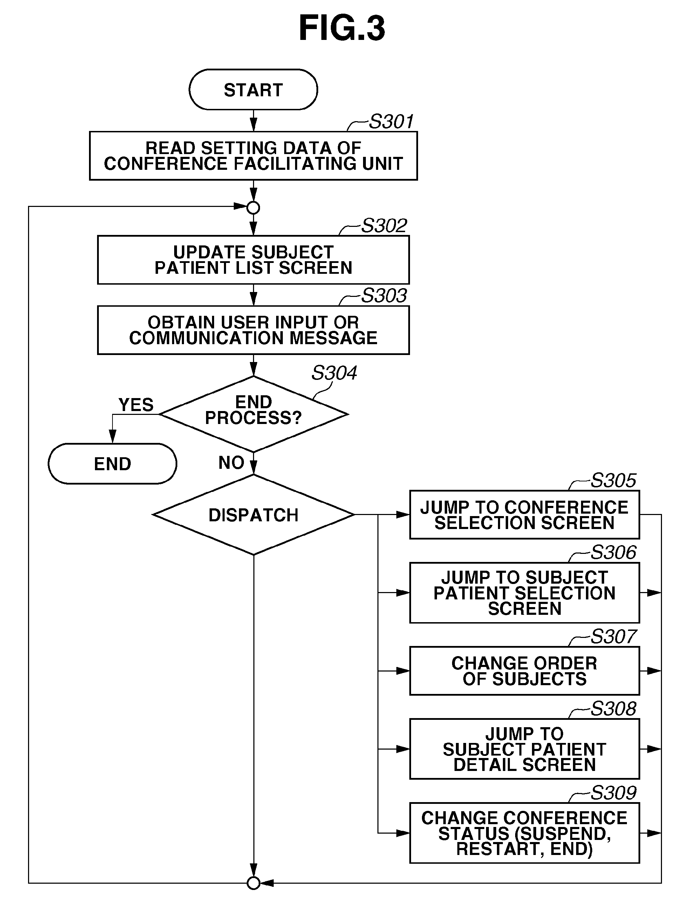

Fig. 3 is a flowchart illustrating an example of a process performed on a subject list screen displayed by a conference facilitating unit in a conference client apparatus 1.

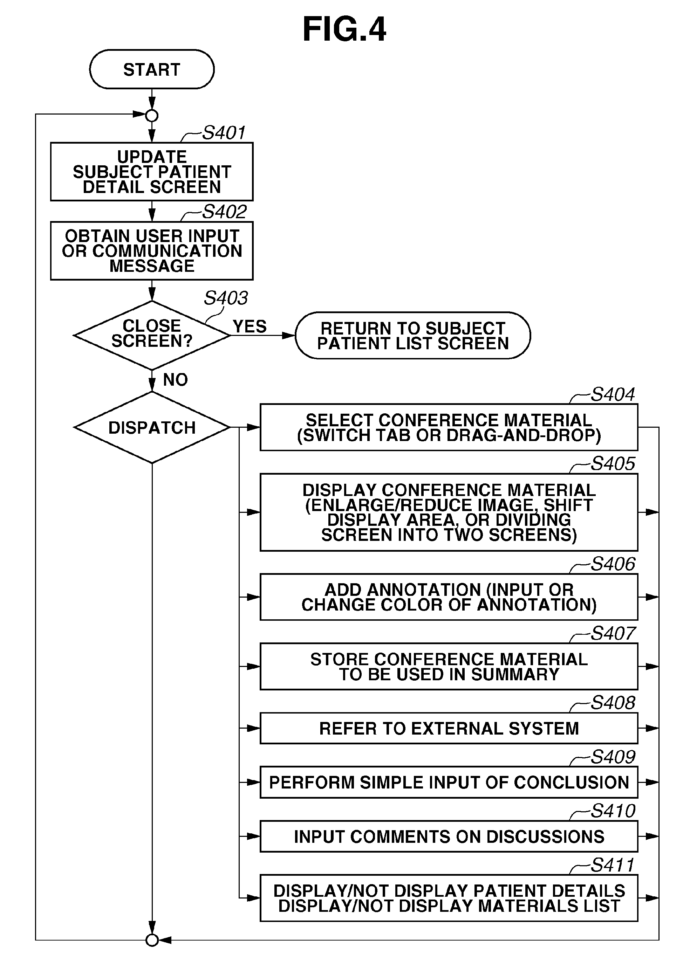

Fig. 4 is a flowchart illustrating an example of a process performed on a subject patient detail screen displayed by the conference facilitating unit in the conference client apparatus 1.

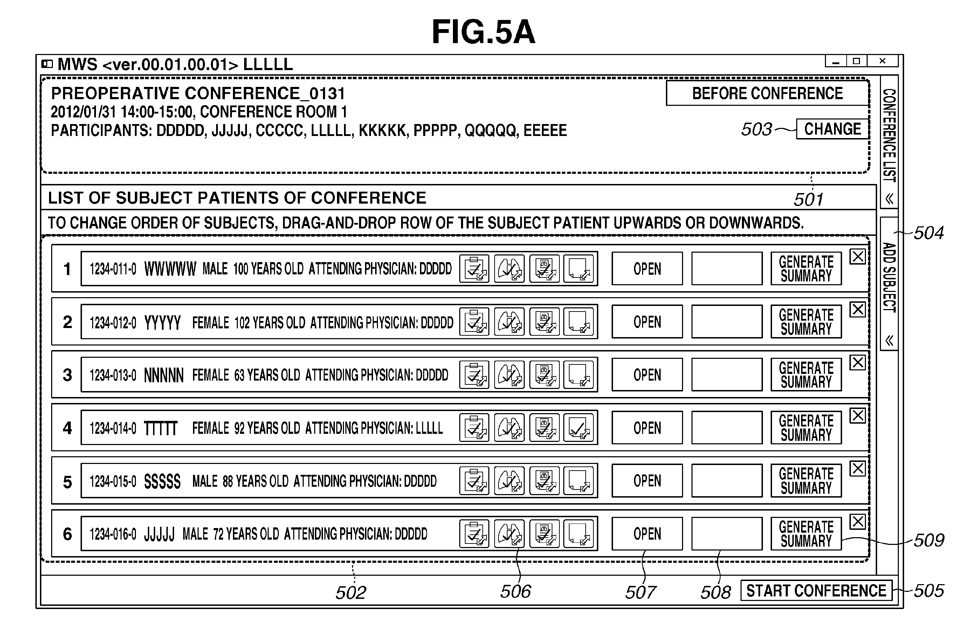

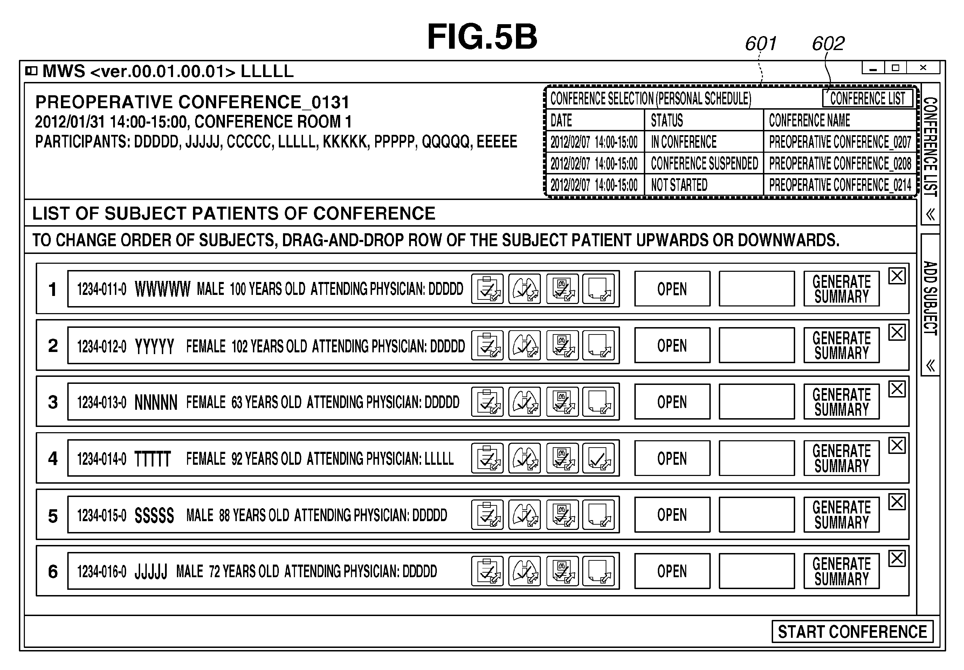

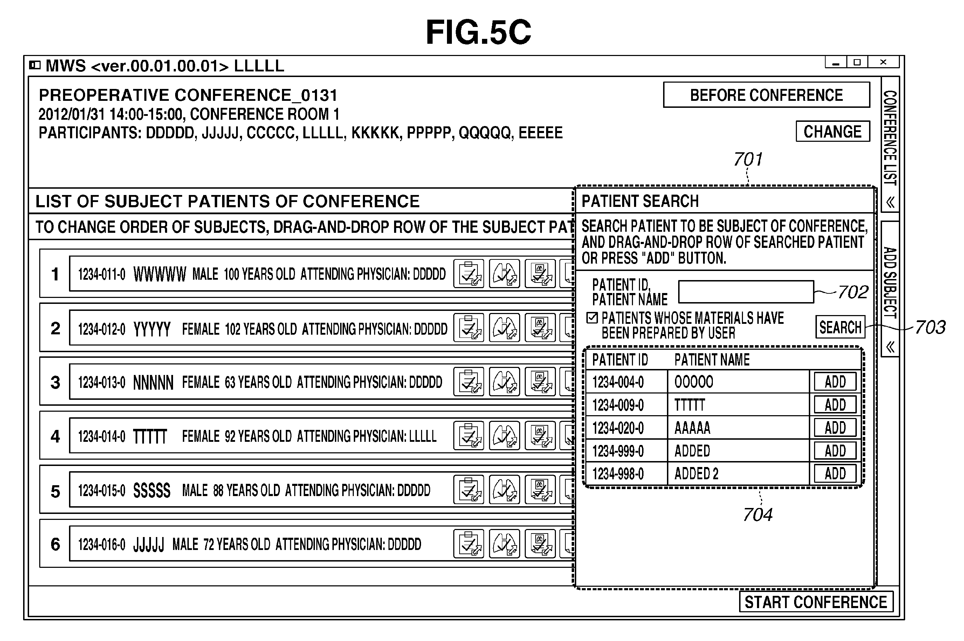

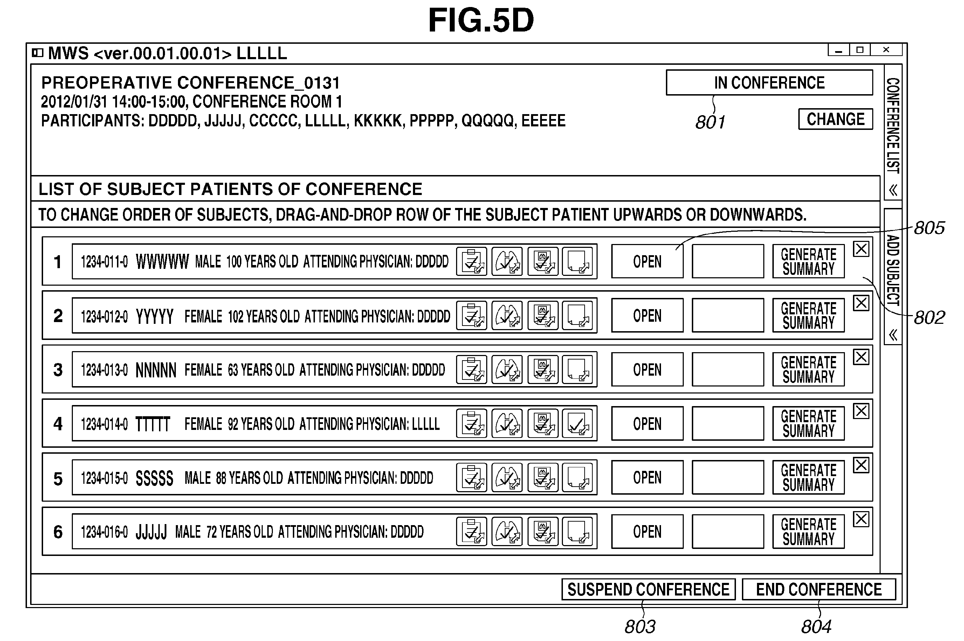

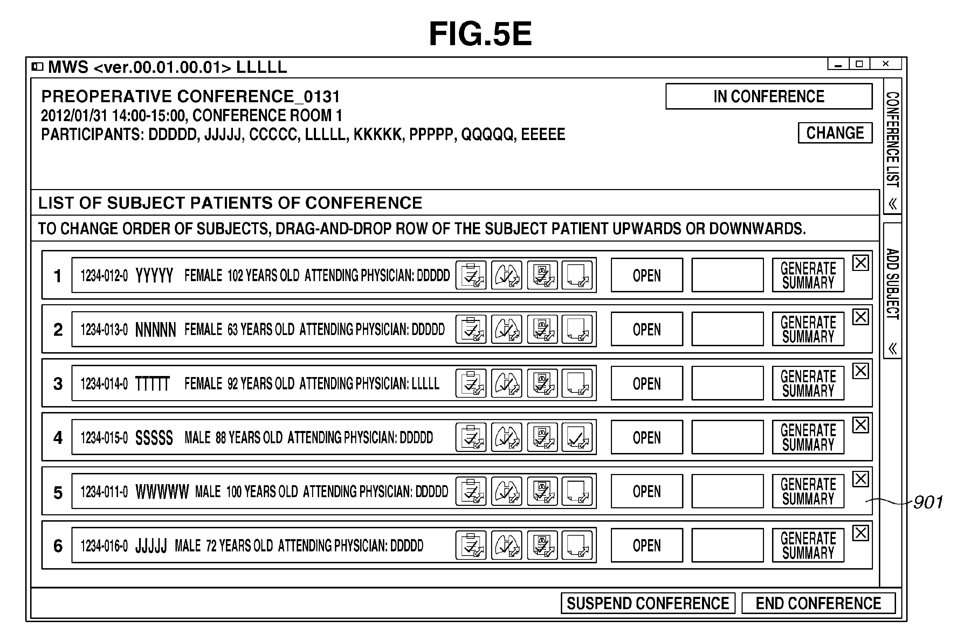

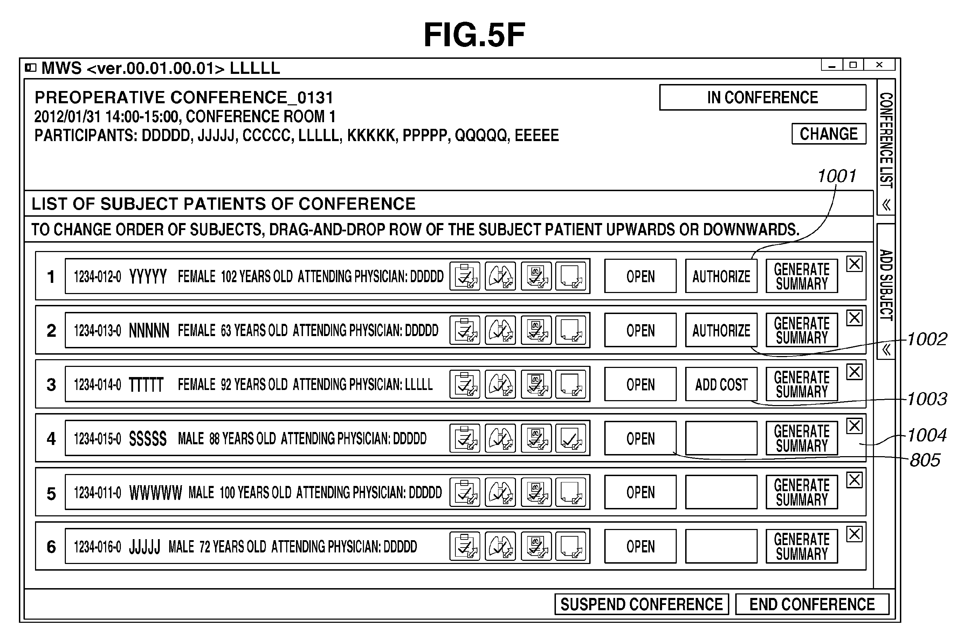

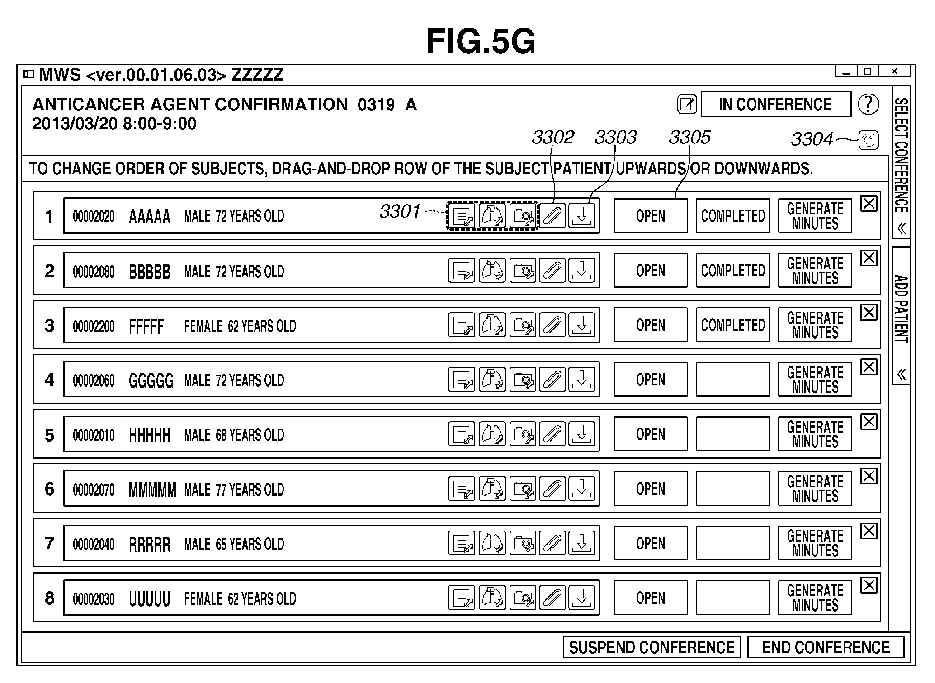

Fig. 5A illustrates an example of a screen displayed by the conference facilitating unit in the conference client apparatus 1 before starting a conference, Fig. 5B illustrates an example of the screen displayed by the conference facilitating unit in the conference client apparatus 1 for performing conference selection before starting the conference, Fig. 5C illustrates an example of the screen displayed by the conference facilitating unit in the conference client apparatus 1 for performing a patient search before starting the conference, Fig. 5D illustrates an example of the screen displayed by the conference facilitating unit in the conference client apparatus 1 when the conference has started, Fig. 5E illustrates an example of the screen in which a subject order has been changed, displayed by the conference facilitating unit in the conference client apparatus 1, Fig. 5F illustrates an example of the screen displayed by the conference facilitating unit in the conference client apparatus 1 before starting the conference on a subject number 4, and Fig. 5G illustrates an example of a different display screen of the patient list displayed by the conference facilitating unit in the conference client apparatus 1.

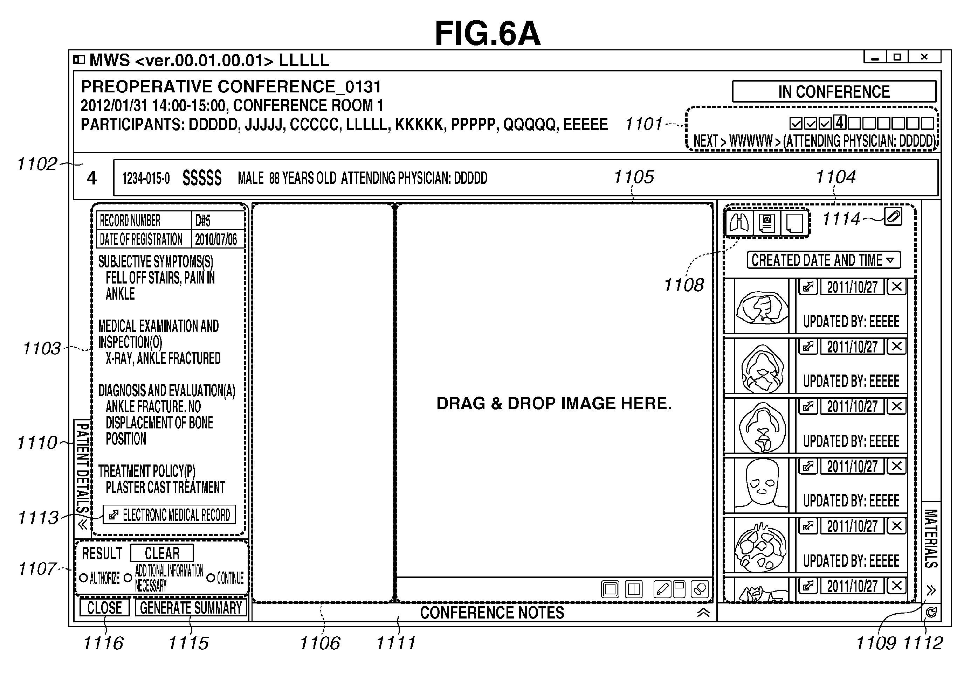

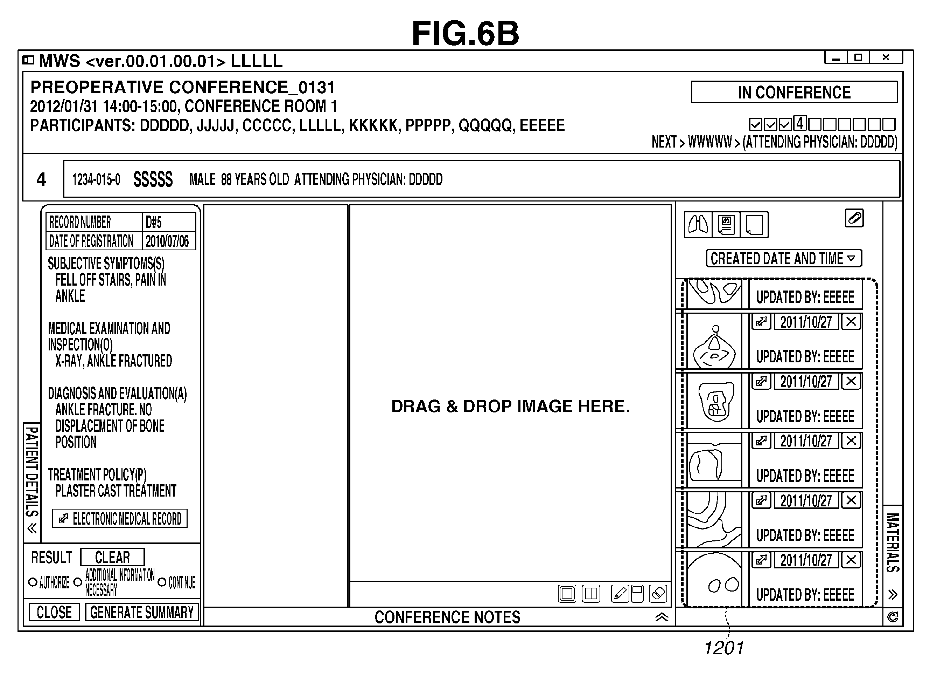

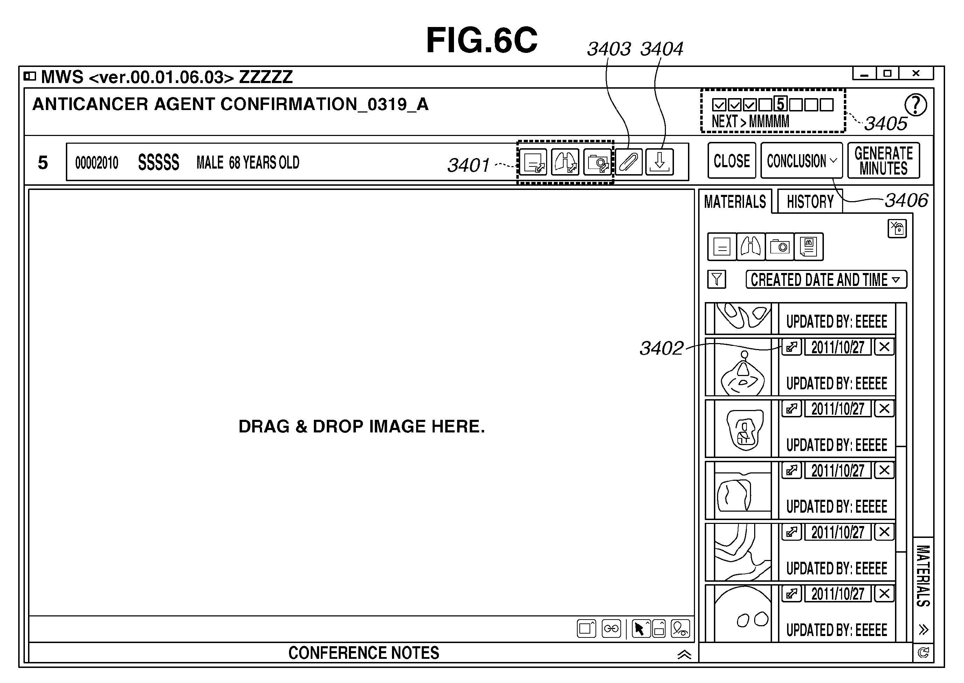

Fig. 6A illustrates an example of a screen displayed by the conference facilitating unit in the conference client apparatus 1 when the conference on the subject number 4 has started, Fig. 6B illustrates an example of the screen displayed by the conference facilitating unit in the conference client apparatus 1 in which a material list (i.e., an examination image tab) has been scrolled, and Fig. 6C illustrates another example of a screen displayed by the conference facilitating unit in the conference client apparatus 1 for displaying patient details.

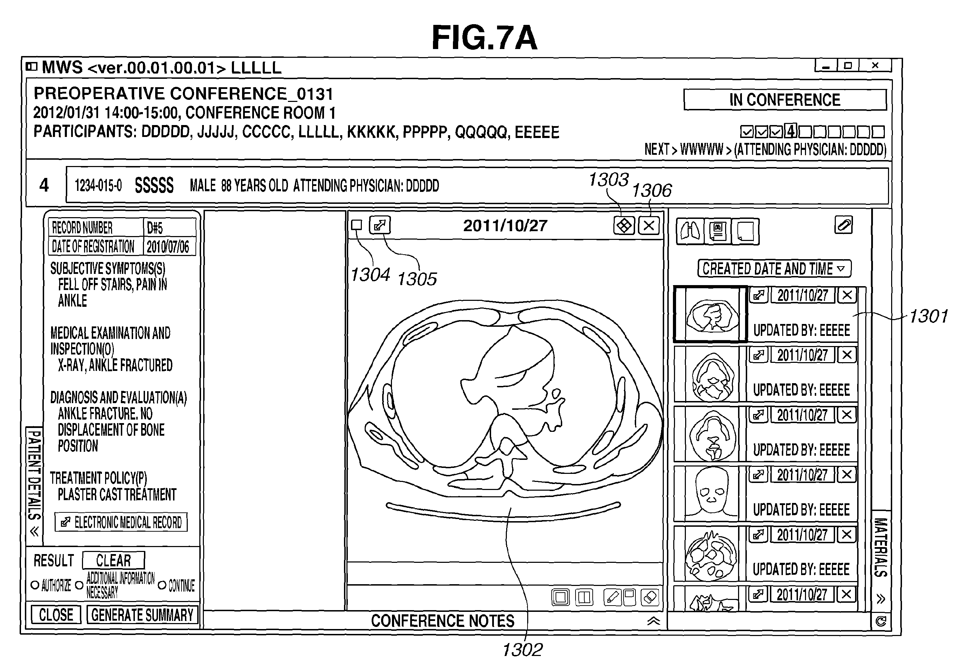

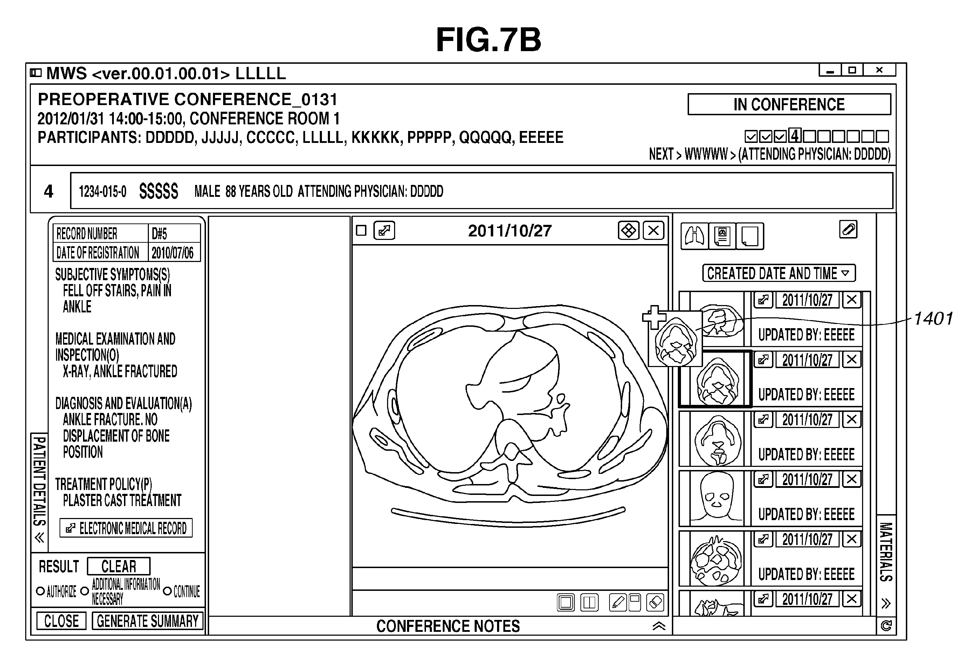

Fig. 7A illustrates an example of a selected material displayed in a workspace on the screen displayed by the conference facilitating unit in the conference client apparatus 1, and Fig. 7B illustrates an example of a drag-and-drop operation of a material from the material list on the screen displayed by the conference facilitating unit in the conference client apparatus 1.

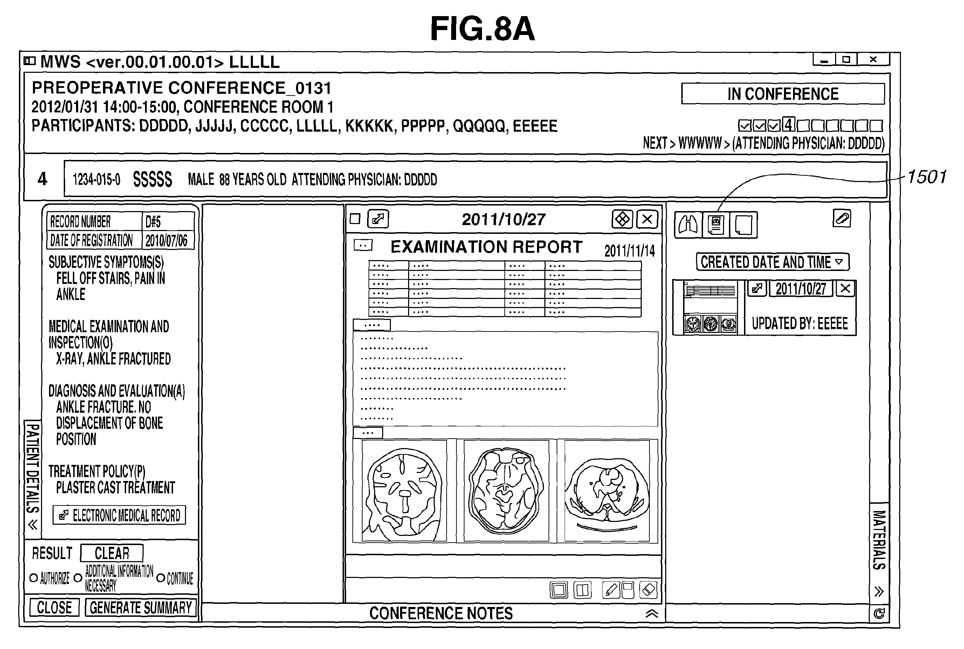

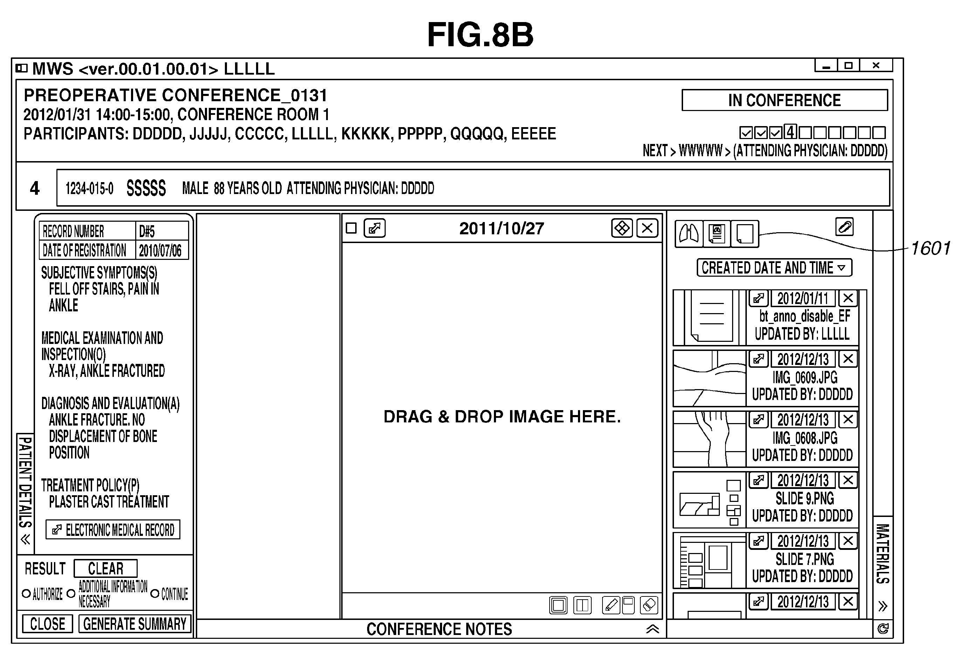

Figs. 8A and 8B illustrate examples of the material list on the screen displayed by the conference facilitating unit in the conference client apparatus 1.

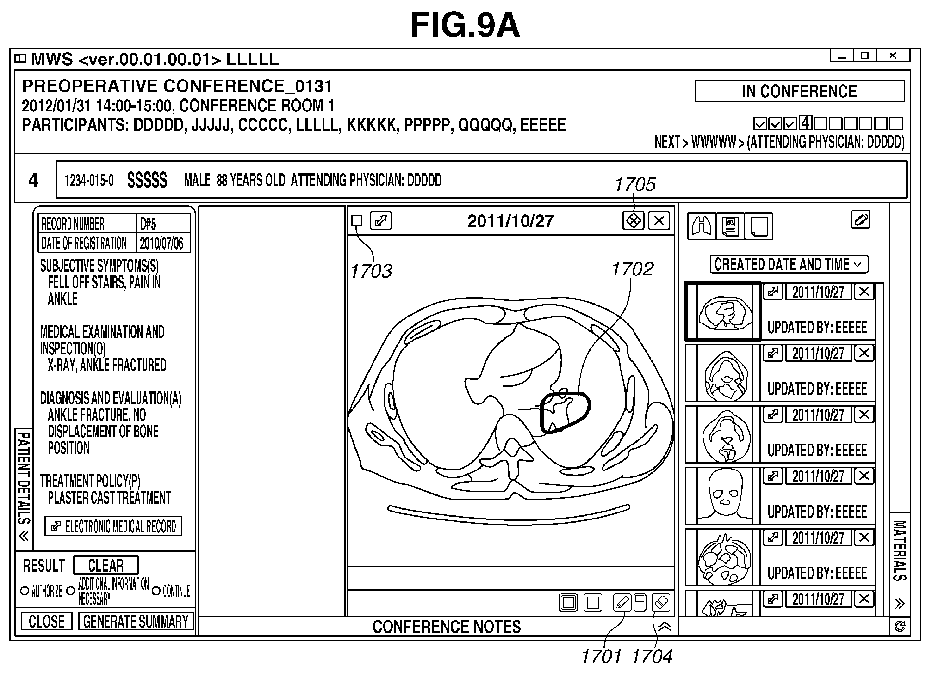

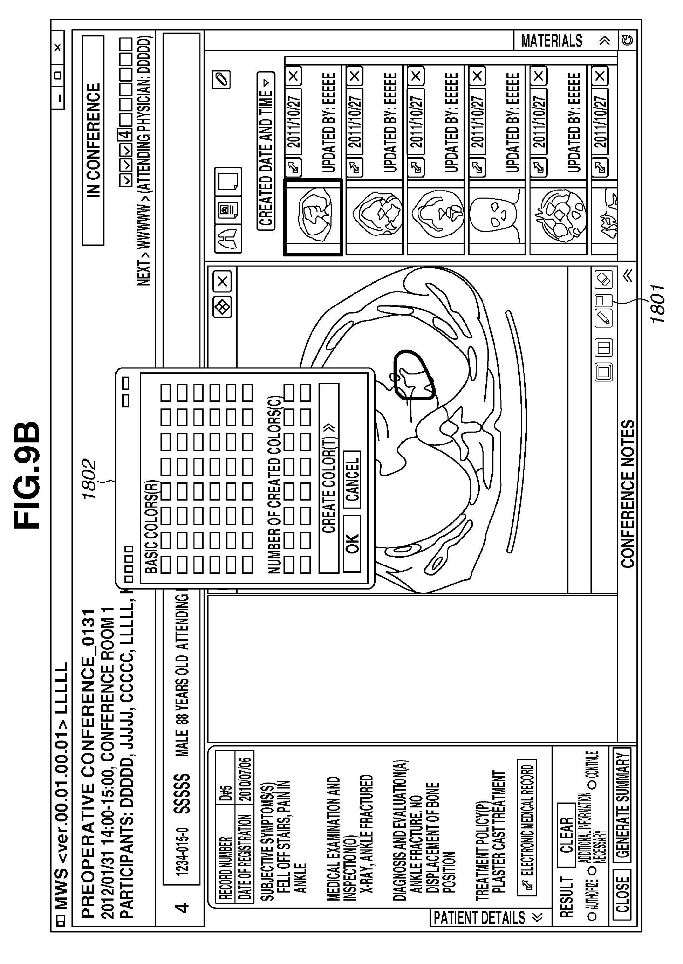

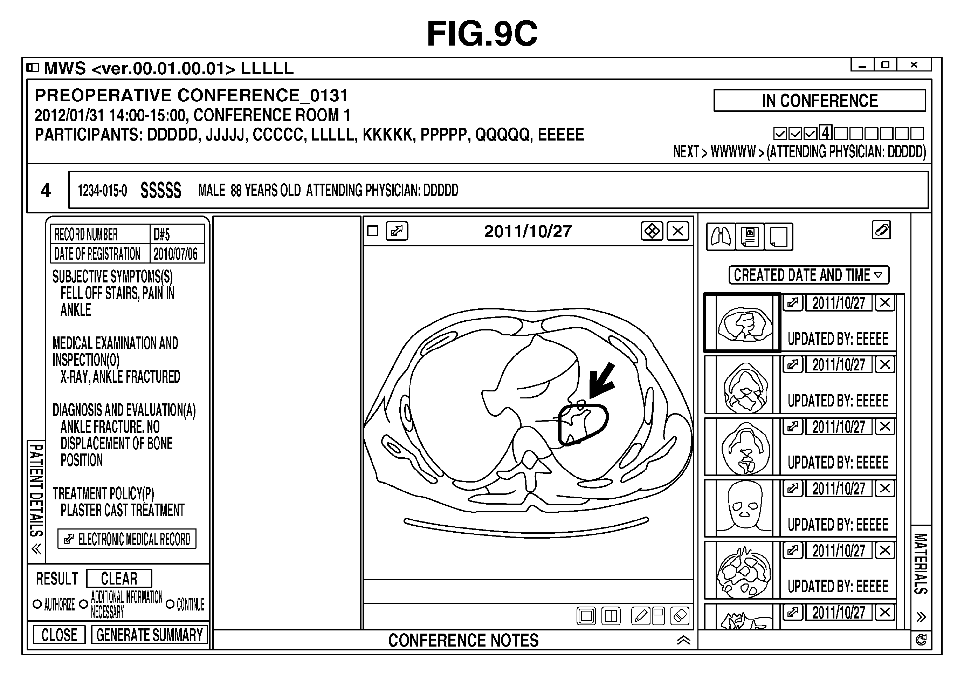

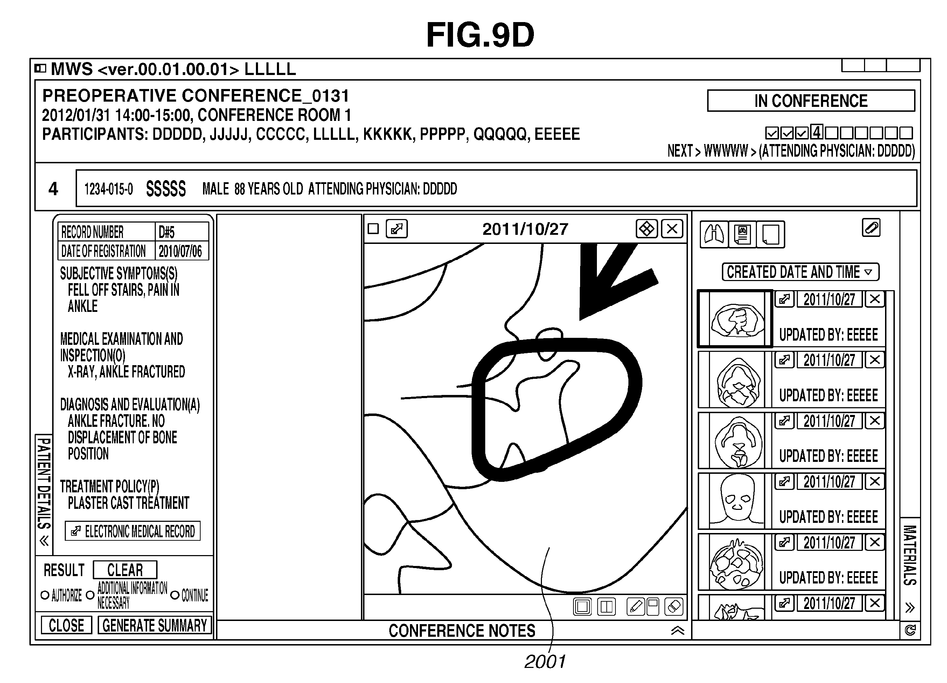

Fig. 9A illustrates an example of an annotation added to a material and an automatic summary setting on the screen displayed by the conference facilitating unit in the conference client apparatus 1, Fig. 9B illustrates an example of a screen for performing annotation color selection displayed by the conference facilitating unit in the conference client apparatus 1, Fig. 9C illustrates an example of an annotation of a different color added on the screen displayed by the conference facilitating unit in the conference client apparatus 1, and Fig. 9D illustrates an example of a scaled display of subject materials on the screen displayed by the conference facilitating unit in the conference client apparatus 1.

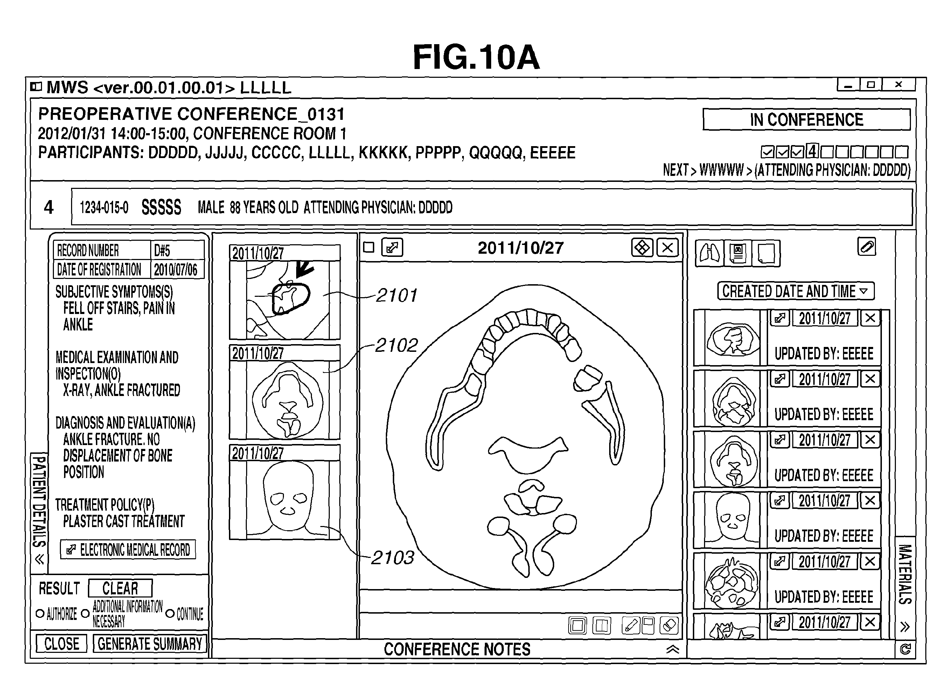

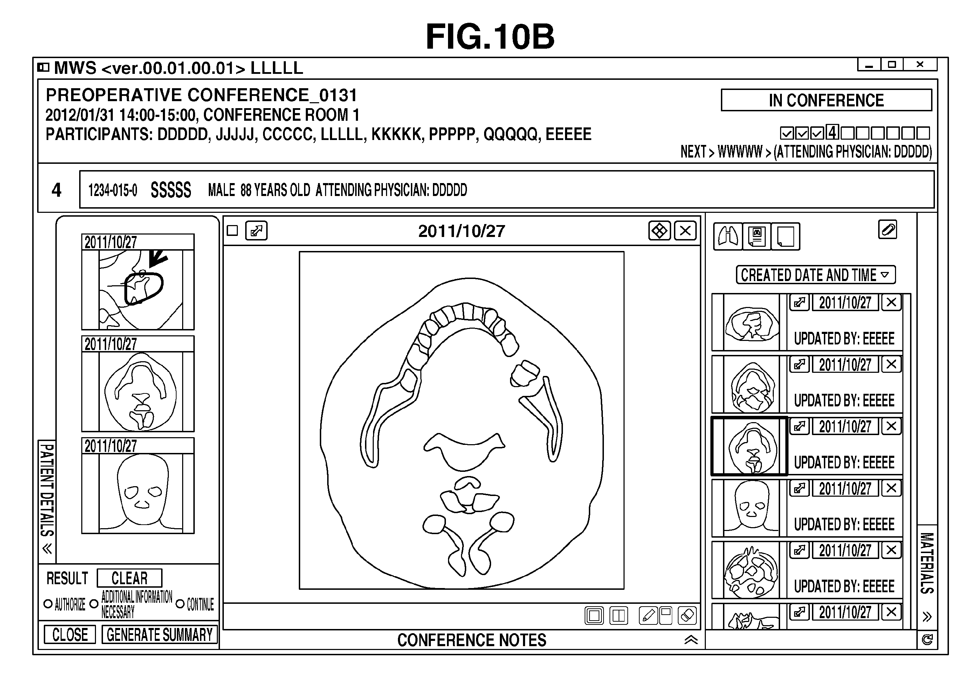

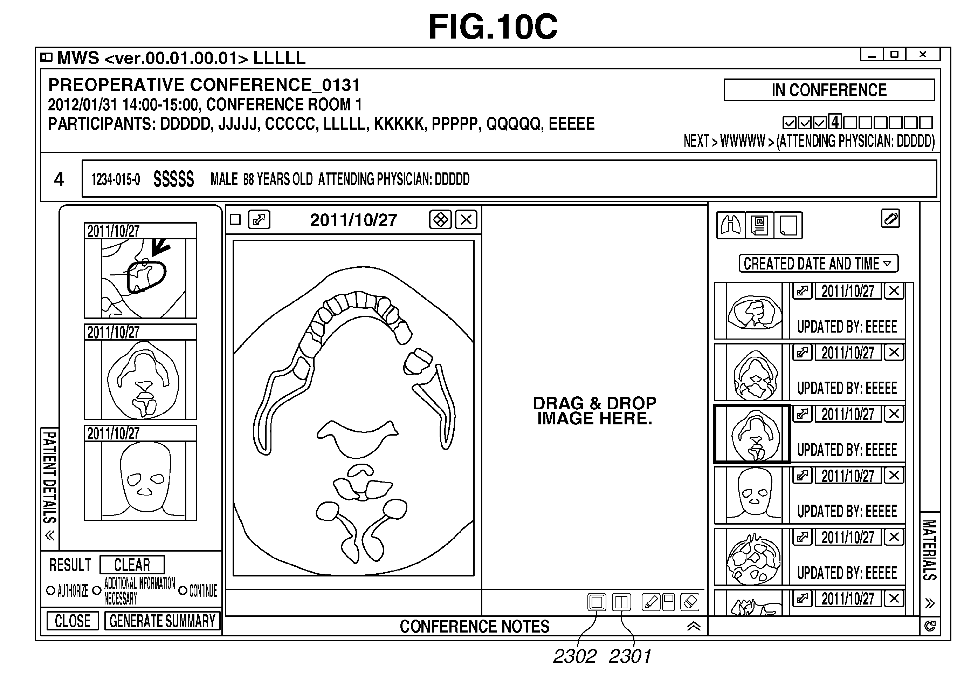

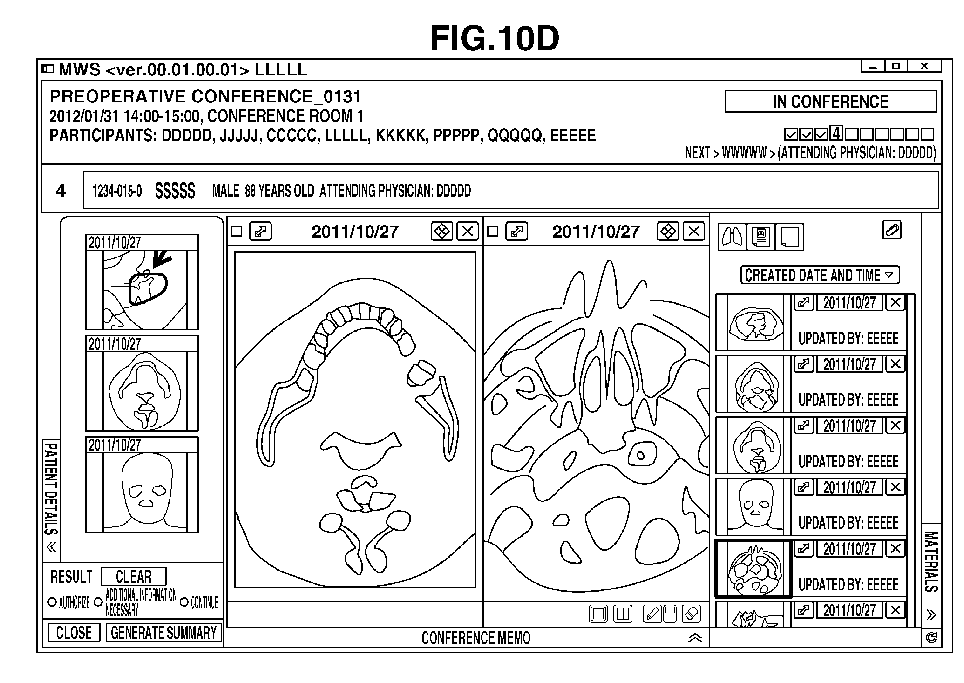

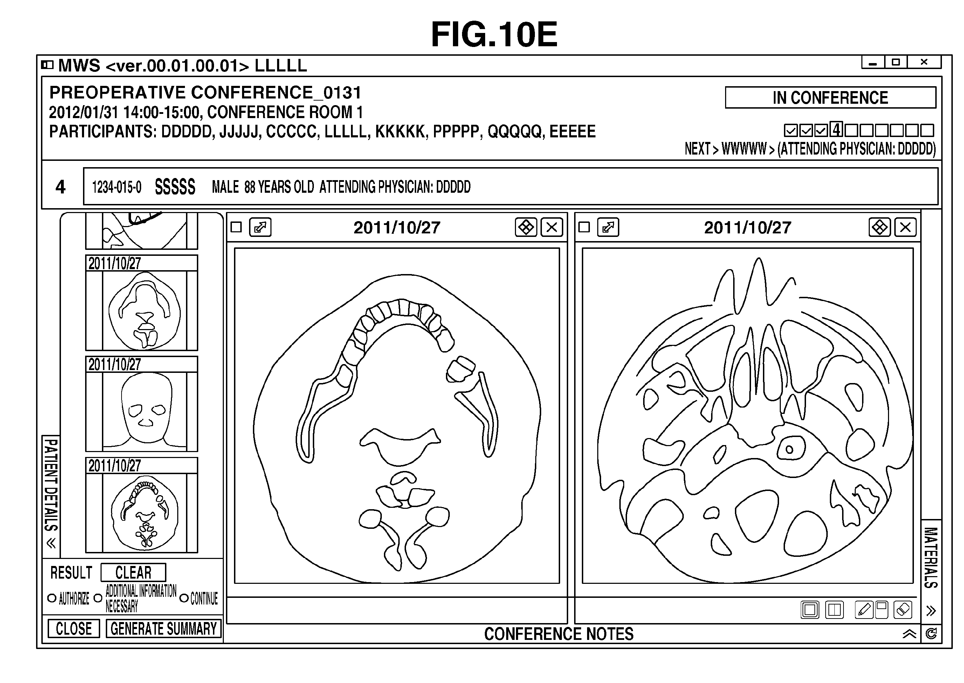

Fig. 10A illustrates an example of the materials set to be used in the summary on the screen displayed by the conference facilitating unit in the conference client apparatus 1, Fig. 10B illustrates an example of not displaying a diagnosis information display area on the screen displayed by the conference facilitating unit in the conference client apparatus 1, Fig. 10C illustrates an example of dividing the screen into 2 screens for displaying the materials on the screen displayed by the conference facilitating unit in the conference client apparatus 1, Fig. 10D illustrates an example of the two-screen display of the materials on the screen displayed by the conference facilitating unit in the conference client apparatus 1, and Fig. 10E illustrates an example of not displaying the material list in the two-screen display of the materials on the screen displayed by the conference facilitating unit in the conference client apparatus 1.

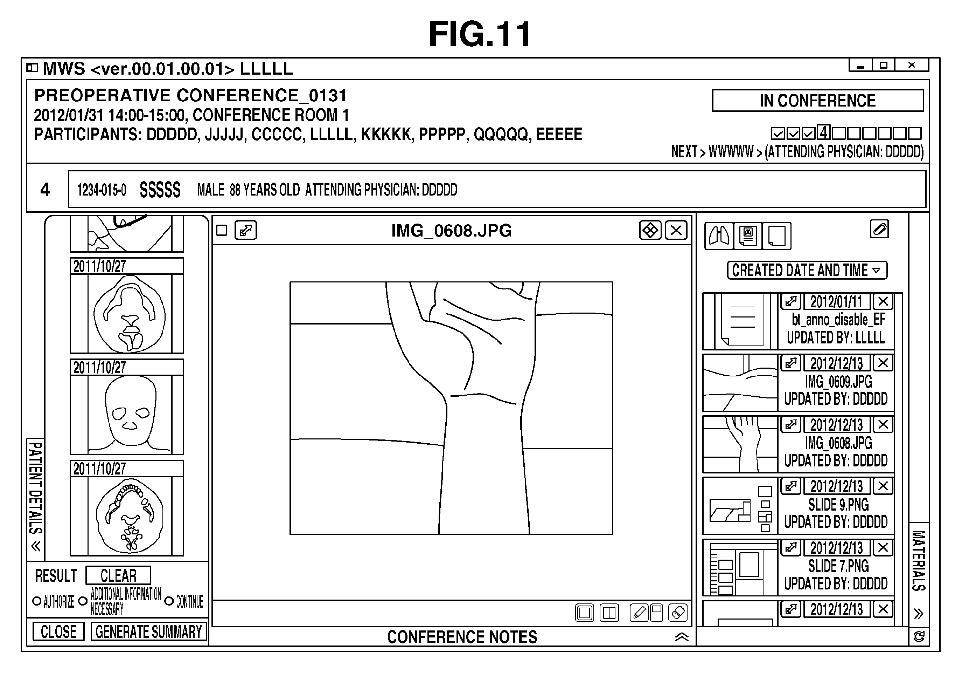

Fig. 11 illustrates an example of displaying the non-DICOM image on the screen displayed by the conference facilitating unit in the conference client apparatus 1.

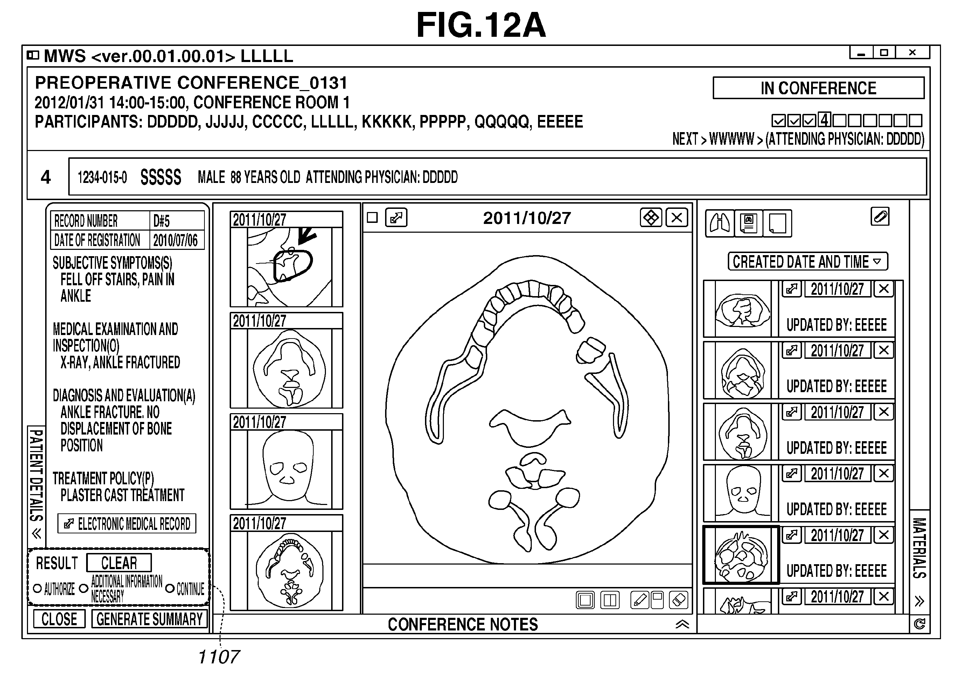

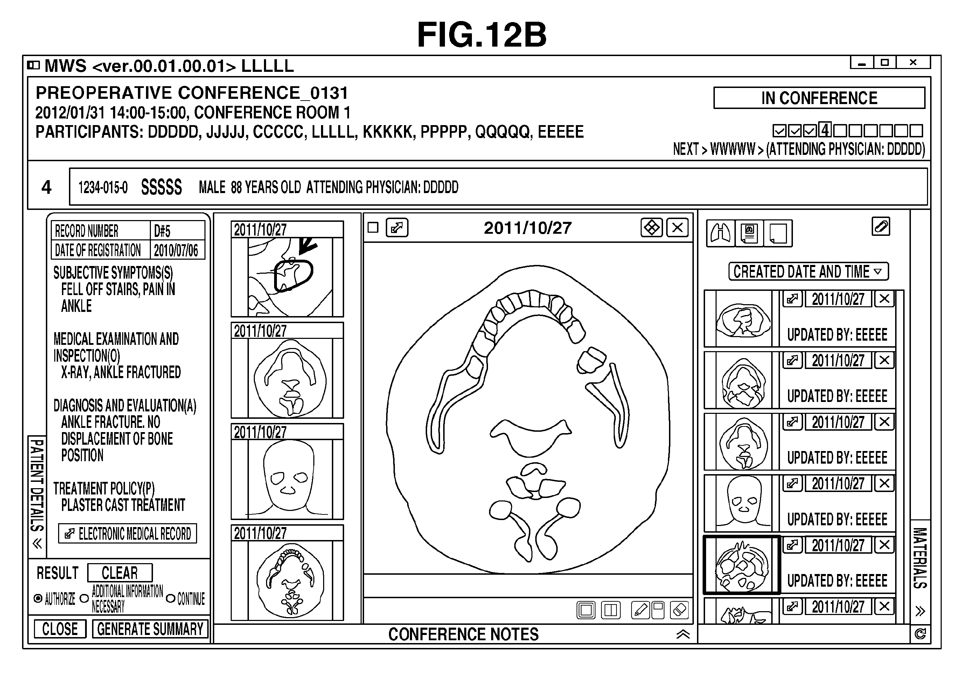

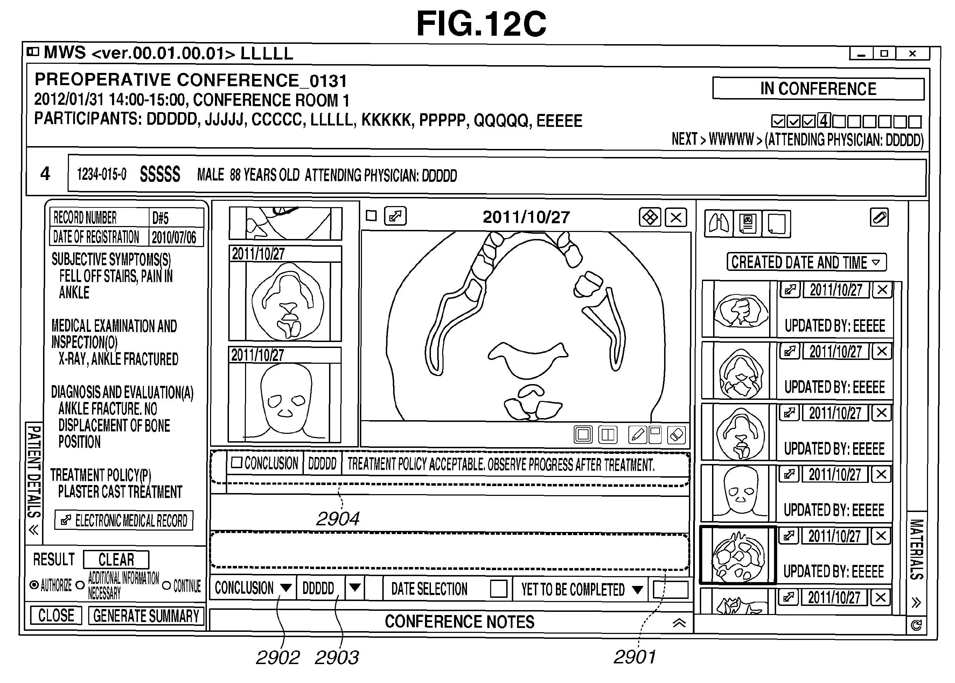

Fig. 12A illustrates an example of an easy input of a conclusion on the screen displayed by the conference facilitating unit in the conference client apparatus 1, Fig. 12B illustrates an example in which the easy input of the conclusion has been performed on the screen displayed by the conference facilitating unit in the conference client apparatus 1, and Fig. 12C illustrates an example of adding a comment on the screen displayed by the conference facilitating unit in the conference client apparatus 1.

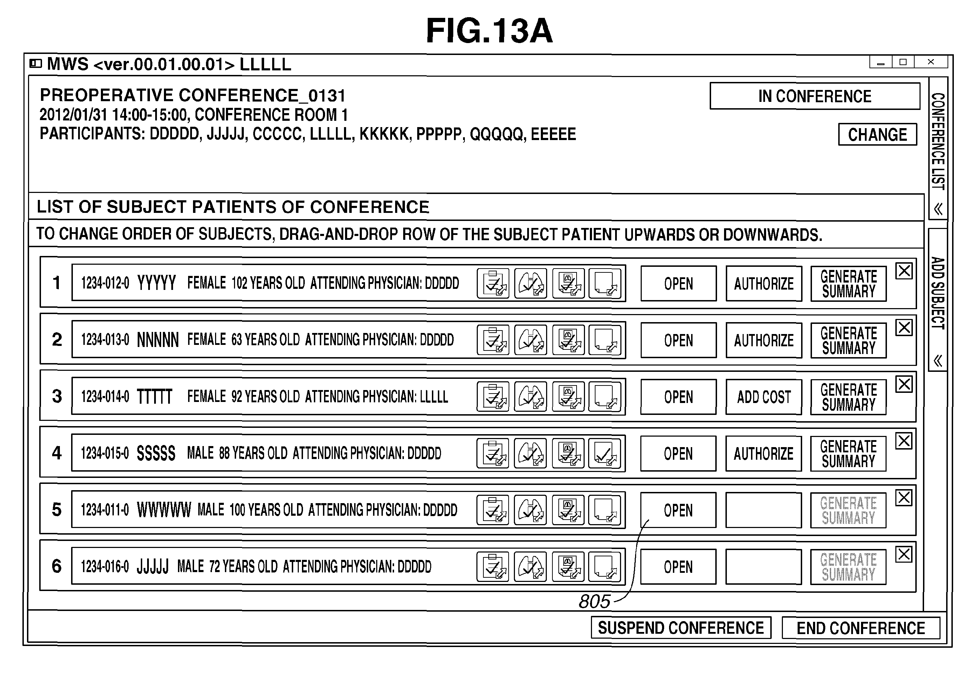

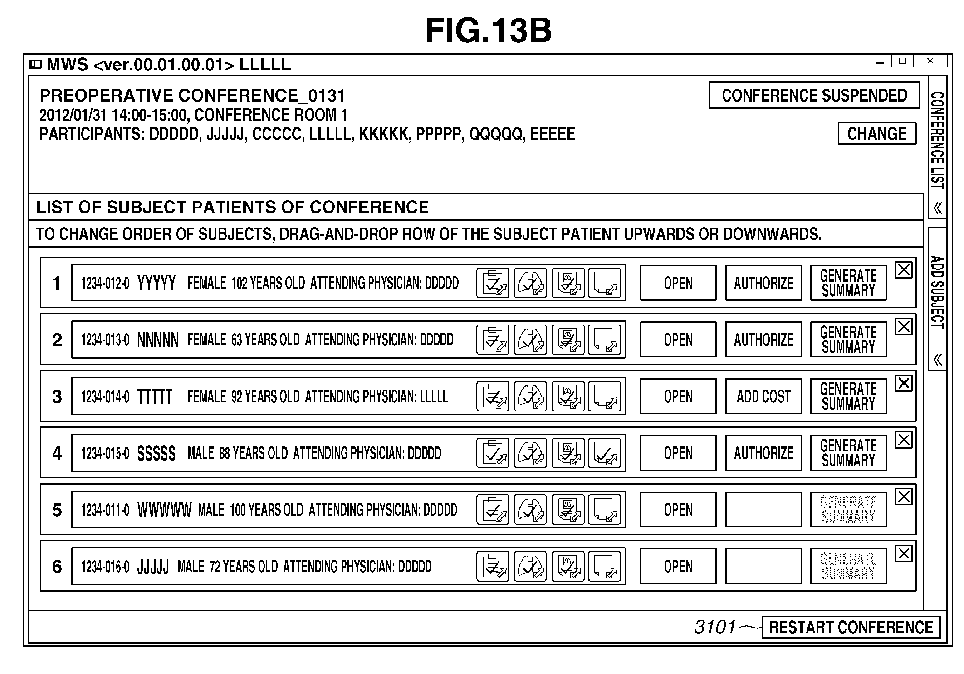

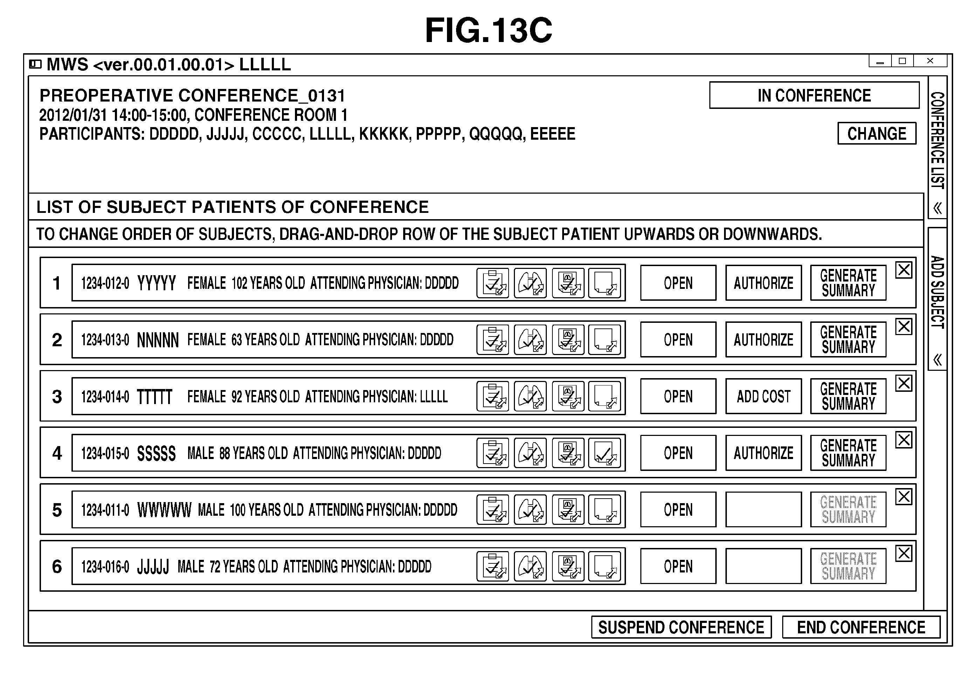

Fig. 13A illustrates an example of the screen displayed by the conference facilitating unit in the conference client apparatus 1 after the conference on the subject has been held and in which the next subject is highlighted, Fig. 13B illustrates an example of the screen displayed by the conference facilitating unit in the conference client apparatus 1 when the conference has been suspended, and Fig. 13C illustrates an example of the screen displayed by the conference facilitating unit in the conference client apparatus 1 when the conference is restarted.

### DESCRIPTION OF THE EMBODIMENTS

Various exemplary embodiments, features, and aspects of the invention will be described in detail below with reference to the drawings. However, configurations described below are merely an example and the present invention is not limited thereto.

According to a first exemplary embodiment of the present invention, a medical conference support system facilitates the conference by collaborating with the electronic medical records, PACS, the examination report system, the image management system, and a file server in the hospital. In particular, according to the present exemplary embodiment, an explicit material preparation trigger employing a material collection button, a relation between the conclusion input and displaying of a conference progress status, and reference to an external system will be described below as the features thereof.

According to the present exemplary embodiment, the conference for discussing, before a respiratory operation (hereinafter referred to as a preoperative conference), symptoms, and the treatment plan of the patient to have the operation will be described below as an example. However, clearly the present exemplary embodiment is not limited thereto. Further, according to the present exemplary embodiment describe below, the preoperative conference is regularly held from 14:00 to 15:00 every Tuesday. Of course, the conference may be at any other time or day and may be irregular.

Fig. 1 illustrates an example of the typical usage according to the exemplary embodiments of the present invention. Referring to Fig. 1, the medical conference support system (i.e., the conference support system) includes a conference client apparatus 1 and a conference server 2. Further, the conference support system is connected to the server group including various servers in the hospital, i.e., a file server 3, a PACS server 4, a diagnosis report server 5, an electronic medical records server 6, an ordering system 7, and an image management server 8. The conference support system may include a plurality of conference client apparatuses 1. Further, functions of the conference server 2 may be included in the conference client apparatus 1, so that the conference support system is of a peer-to-peer (P2P) configuration. The conference client apparatus 1, the conference server 2, and the group of various servers (servers 3 to 8) in the hospital are connected by a wired or a wireless network 9.

Since hardware configurations of the conference client apparatus 1 and the conference server 2 are similar, the hardware configuration of the conference client apparatus 1 will be described in detail. A user interface (UI) device 101, such as a mouse, a digitizer, or a keyboard, is used for inputting an instruction from a user to the conference client apparatus 1. A central processing unit (CPU) 102 interprets and executes the program read from a program storage area 106 to a random access memory (RAM) 103. As a result, various controls and calculations can be performed in the apparatus, and the UI can be displayed. A communication interface (IF) 104 connected to the network 9 functions as a communication interface between the conference server 2 and the various servers (i.e., the file server 3, the PACS server 4, the diagnosis report server 5, the electronic medical records server 6, the ordering system 7, and the image management server 8) in the hospital. A UI display unit 105 such as a light-emitting diode (LED) or a liquid crystal display panel displays the status of the apparatus and processing contents. Further, the conference client apparatus 1 includes the program storage area 106 and a data storage area 107. More specifically, the program storage area 106 and the data storage area 107 can be realized using a hard disk and a flash memory. However, the present invention is not dependent on a specific storage medium. The data storage area 107 in the conference client apparatus 1 stores conference materials 10. The file server 3 may store the conference materials 10 instead of the data storage area 107. A data storage area 207 in the conference server 2 stores material registration information 20 and conference information 21.

The conference materials are collected for each patient or for each case, and managed in units of binders. More specifically, the actual contents of the conference materials are not stored in the binder, and only management information of the conference materials is stored in the binder. A binder pool 301 is arranged in the file server 3, and a binder 302 for each patient or for each case is generated therein. The binder pool 301 may be arranged in the data storage area 207 of the conference server 2. Presentation images 30 and thumbnails 31 are generated for each registered material in the binder 302. Since a size of the medical image is large and thus difficult to handle, the presentation images 30 appropriate for display in the conference are generated. Further, the information on a note which has been handwritten on the image during the conference is stored as an annotation 32 in each binder 302. Furthermore, the conclusion of the conference and the images used in the conference are collected into one document as a summary 33 and stored in each binder 302.

The medical conference support system configured as described above operates on the conference client apparatus and the conference server apparatus. The medical conference support system thus collaborates with the electronic medical records and PACS connected via the network, and facilitates the conference.

The network illustrated in the drawings may be an intranet managed in the hospital or an organization, or the Internet. Further, the network may be wireless or wired.

Furthermore, the electronic medical records server, PACS, the examination report system, the image management server, the file server, and the ordering system are widely-used apparatuses. The hardware configuration examples and description of operation flows thereof will thus be omitted.

Fig. 2 is a schematic diagram illustrating a program configuration of the conference support apparatus. Referring to Fig. 2, the conference client apparatus 1 includes a conference preparation unit, the conference facilitating unit, and a conference summary output unit. The conference preparation unit is a unit for preparing the conference materials such as patient examination data and sets the subject of the conference. The conference facilitating unit is a unit for referring to the conference materials of the set subject during the conference and facilitating the conference. The conference summary output unit is a unit for generating the conference result as the summary and distributing the summary. The programs are stored in a memory or a storage device in each apparatus and executed by the CPU in each apparatus. The programs then function as necessary by controlling the network I/F and a universal serial bus (USB) I/F, or the various controllers. Further, the programs detect a user operation or an automatic operation, and perform the processes by receiving the following commands and operations: a communication command via the network I/F, the operation of the mouse, the keyboard, or a remote controller via a peripheral controller, or a voice command via a microphone input.

The above-described configuration according to the present exemplary embodiment enables the medical conference support system to collaborate with the electronic medical records server, PACS, the examination report system, the image management server, and the file server and facilitate the conference. In particular, according to the present exemplary embodiment, the explicit material preparation trigger employing the material collection button, the relation between the conclusion input and displaying the conference progress status, and reference to the external system will be described below as the features thereof.

The processes performed by each of the programs will be described below with reference to the drawings. According to the following descriptions on the program operations, the setting information read at activation of each program is read from a non-volatile storage device or the network when each apparatus is started. Each of the read setting information is stored in the memory or the storage device in each apparatus. Setting values thereof are default values set at the time of factory shipment, or user-designated values set using a different tool.

Fig. 3 is a flowchart illustrating a process performed on the subject list screen displayed by the conference facilitating unit in the conference client apparatus 1. The conference facilitating unit displays the conference information on a selected conference and a list of the patients to be the subject of the conference in the subject list screen. The conference facilitating unit then receives an operation from the user (i.e., the physician or a medical staff), and performs processing according to the operation.

In step S301, the conference facilitating unit reads the setting information thereof. In step S302, the conference facilitating unit updates the subject patient list screen. In step S303, the conference facilitating unit obtains the user input or a communication message. The conference facilitating unit then performs, according to the content of the input or the communication message, processing as described below. If the conference facilitating unit determines in step S304 that an end instruction has been received (YES in step S304), the conference facilitating unit ends the process. If the conference facilitating unit determines otherwise (NO in step S304), the conference facilitating unit performs processes as follows. If a conference selection instruction has been received, the process proceeds to step S305. In step S305, the conference facilitating unit causes the screen to jump to a conference list screen (as illustrated in Fig. 5B to be described below), and stands by for the next instruction. If a subject patient selection instruction has been received, the process proceeds to step S306. In step S306, the conference facilitating unit causes the screen to jump to a patient search screen (as illustrated in Fig. 5C to be described below) for selecting the patient, and stands by for the next instruction. If a subject order change instruction has been received, the process proceeds to step S307. In step S307, the conference facilitating unit changes the order of the patients as instructed on the subject patients list screen (as illustrated in Fig. 5E to be described below), and stands by for the next instruction. If a subject patient details instruction has been received, the process proceeds to step S308. In step S308, the conference facilitating unit causes the screen to jump to a subject patient detail screen (as illustrated in Fig. 6A to be described below), and stands by for the next instruction. If a conference status change instruction has been received, the process proceeds to step S309. In step S309, the conference facilitating unit changes the conference status as instructed on the subject patients list screen (as illustrated in Fig. 5D, Fig. 13B, and Fig. 13C to be described below), and stands by for the next instruction. After performing the processes of step S305 to step S309, the process returns to step S302, and the conference facilitating unit repeats the above-described processes.

Fig. 4 is a flowchart illustrating a process performed on the subject patient detail screen displayed by the conference facilitating unit in the conference client apparatus 1. The conference facilitating unit displays the examination images, various reports, and the non-DICOM images of the patient selected as the subject, on the subject patient detail screen the diagnosis information. The conference facilitating unit then receives the operation from the user (i.e., the physician or the medical staff) on the subject patient detail screen, and performs processing according to the operation.

In step S401, the conference facilitating unit updates the subject patient detail screen. In step S402, the conference facilitating unit obtains the user input or the communication message. The conference facilitating unit then performs processing according to the content of the input or the communication message. If the conference facilitating unit determines in step S403 that a "close" instruction has been received (YES in step S403), the conference facilitating unit closes the subject patient detail screen, and returns to the subject patients list screen. If the conference facilitating unit determines otherwise (NO in step S403), the conference facilitating unit performs processes as follows. If a conference material selection instruction has been received, the process proceeds to step S404. In step S404, the conference facilitating unit selects the designated material, and stands by for the next instruction. Such a conference material selection instruction includes switching of the conference materials tab and performing the drag-and-drop operation on the conference material to the workspace (as illustrated in Figs. 6B, 7A, 7B, 8A, and 8B to be described below). If a conference material display instruction has been received, the process proceeds to step S405. In step S405, the conference facilitating unit displays the conference material, and stands by for the next instruction. Such a conference material display instruction includes enlarging and reducing the conference material in the workspace, moving a display area, and dividing the screen into 2 screens (as illustrated in Figs. 9D, 10C, and 10D to be described below). If an annotation instruction has been received, the process proceeds to step S406. In step S406, the conference facilitating unit adds the annotation to the conference material in the workspace, and stands by for the next instruction. Such an annotation instruction includes inputting the annotation and changing the color of the annotation (as illustrated in Figs. 9A, 9B, and 9C to be described below). If a summary material storing instruction has been received, the process proceeds to step S407. In step S407, the conference facilitating unit stores the conference material in the workspace to be used in the summary, and stands by for the next instruction. Such a summary material storing instruction includes specifying an automatic summary setting and displaying the materials to be used in the summary (as illustrated in Figs. 9A and 10A to be described below). If an external system reference instruction has been received, the process proceeds to step S408. In step S408, the conference facilitating unit calls the external system such as the electronic medical records and PACS, and stands by for the next instruction. If an instruction for performing easy input of the conclusion has been received, the process proceeds to step S409. In step S409, the conference facilitating unit inputs the conclusion of the discussions on the diagnosis and observations and the treatment plan of the subject patient (as illustrated in Fig. 12B to be describe below), and stands by for the next instruction. If a proceedings comment input instruction has been received, the process proceeds to step S410. In step S410, the conference facilitating unit inputs the comment with respect to the discussions on the subject patient (as illustrated in Fig. 12C to be described below), and stands by for the next instruction. If an instruction to display/not display the patient details or the material list has been received, the process proceeds to step S411. In step S411, the conference facilitating unit switches between displaying/not displaying the patient details and the material list on the screen (as illustrated in Figs. 10B and 10E to be described below), and stands by for the next instruction. After the conference facilitating unit performs the processes of step S404 to step S411, the process returns to step S401, and the conference facilitating unit repeats the above-described processes.

The screens displayed on the UI display unit by the conference facilitating unit in the conference client apparatus 1 will be described below. Such screens are displayed on the UI display unit 105 (i.e., the display controller) in the conference client apparatus 1 and operated by the input using the UI device 101.

Fig. 5A illustrates a screen displayed by the conference facilitating unit in the conference client apparatus 1 before starting the conference. The conference client apparatus 1 obtains the necessary information from the conference server 2, and automatically displays the latest conference in which the user (i.e., the physician or the medical staff member) of the conference client apparatus 1 is set as a participant. Referring to Fig. 5A, the screen includes a conference status display area 501. A conference name set in advance, a conference date and time, a conference location, and a list of participants are displayed in the left portion of the conference status display area 501. According to the present exemplary embodiment, the following values are displayed with respect to the preoperative conference of the respiratory operation. "Preoperative conference_0131"
"2012/01/31 14:00-15:00"
"Conference room 1"
"Participants: DDDDD, JJJJJ, CCCCC, LLLLL, KKKKK, PPPPP, QQQQQ, EEEEE"

Further, a conference status (i.e., before conference in this example) and a change button are displayed in the right portion of the conference status display area 501. If the user presses the change button, the conference preparation unit in the conference client apparatus 1 is called, and the conference name, the conference date and time, the conference location, and the participants become changeable. A subject patient list display area 502 displays the list of the patients preset as the subjects by the conference preparation unit. For example, a conditional expression for automatically extracting the patient to be the subject of the conference is set for each conference (i.e., the respiratory unit preoperative conference according to the present exemplary embodiment). The conference preparation unit then automatically extracts the patient two hours before starting the conference (12:00 on January 31 according to the present exemplary embodiment), so that the patient is set as the subject of the conference.

More specifically, the condition for extracting the subject patient may be set as follows.

### [The condition for automatically extracting the subject patient of the respiratory unit preoperative conference]

The patient whose operation scheduled date set according to an operation order issued from the physician of the medical department is within one week from the conference date, is extracted as the subject patient. The patient is extracted as described above for discussing the plans on the operations scheduled up to the next conference (next week) in the preoperative conference held every week, by considering the opinions of other physicians participating in the conference.

The patient information for each of the patients in the patient list includes patient basic information (i.e., patient identification (ID), name, sex, and age), the name of the physician in charge, material preparation status icons, a subject display button, a conclusion display area, and a summary generation button.

For example, according to the present exemplary embodiment, the patient list as described below is displayed with respect to the respiratory unit preoperative conference.
"1234-011-0 WWWWW male 100 years old physician: DDDDD"
"1234-012-0 YYYYY female 102 years old physician: DDDDD"
"1234-013-0 NNNNN female 63 years old physician: DDDDD"
"1234-014-0 TTTTT female 92 years old physician: LLLLL"
"1234-015-0 SSSSS male 88 years old physician: DDDDD"
"1234-016-0 JJJJJ male 72 years old physician: DDDDD"
Attribute values derived from the extraction condition of the subject patient other than those described above may also be displayed.

For example, the operation scheduled date, a surgeon of the operation, or an operation method may also be displayed.

If the user presses a conference list button 503, the screen jumps to the screen illustrated in Fig. 5B to be described below. If the user presses a patient selection button 504, the screen jumps to the screen illustrated in Fig. 5C to be described below. If the user presses a conference start button 505, the screen jumps to the screen illustrated in Fig. 5D to be described below. Material preparation status icons 506 display the preparation statuses of the materials with respect to each of the subject patients included in the subject patient list display area 502. According to the present exemplary embodiment, the material preparation status icons 506 indicate, from the left, the preparation statuses of medical record information (e.g., the diagnosis and observations and the treatment plan), examination image information (e.g., the DICOM image), report information (e.g., a radiologic interpretation report or a pathological diagnosis report), and other information (e.g., a camera image or a scheme figure). The material preparation status icons 506 thus display to be easily understandable the preparation status of each type of conference material with respect to the patient.

Further, each of the material preparation status icons 506 also function as the external system call button for preparing each type of conference material. More specifically, the medical record information icon activates the electronic medical records, the examination image information icon activates a PACS viewer, the report information icon activates a report viewer, and the icon for other information activates a file browser. If the user presses a subject display button 507 indicating "open", the screen jumps to the screen illustrated in Fig. 6A to be described below. A conclusion display area 508 and a summary generation button 509 are also included in the subject patient list display area 502. If the user presses the summary generation button 509, the conference summary output unit is called.

Fig. 5B illustrates a screen displayed by the conference facilitating unit in the conference client apparatus 1 for performing conference selection before starting the conference. Referring to Fig. 5B, if the user presses the conference list button 503 on the screen illustrated in Fig. 5A, a conference selection panel 601 is slid in from the right and displayed. The conference selection panel 601 displays the list of conferences the user is assigned to participate in. If the user selects a desired conference from the list, the screen switches from the conference status to the subject patient list in the screen. A conference list button 602 is displayed in the upper portion of the conference selection panel 601. If the user presses the conference list button 602, the conference list in the conference preparation unit is displayed, and the user can switch the conference. The user can select a conference from the displayed conference list, by using a pointing device such as the mouse or a touch panel. In other words, the conference list button 602 corresponds to an example of the selection unit configured to select a conference from the plurality of conferences.

Fig. 5C illustrates a screen displayed by the conference facilitating unit in the conference client apparatus 1 for performing the patient search before starting the conference. Referring to Fig. 5C, if the user presses the patient selection button 504 on the screen illustrated in Fig. 5A, a patient selection panel 701 is slid in from the right and displayed. The user can then search for the desired patient using the patient selection panel 701 and add the patient to the subject patient list display area 502. An input area 702 is for inputting the patient name and the patient ID, and thus allows designation of the condition for searching for the desired patient. The conference client apparatus 1 then searches for the patient whose patient ID and name partially match the input character string. Further, there is a check box below the input area 702 for limiting the patients to be searched to the patients the user has prepared the materials thereof. If the user presses a search button 703, the patients matching the search condition are displayed as a list.

A search result list 704 is the list displaying the patients matching the search condition. An add button is displayed for each item in the search result list 704. If the user presses the add button, the corresponding patient is added to the end portion of the subject patient list display area 502. Further, if the user directly drags and drops the patient from the search result list 704 to the subject patient list display area 502, the patient is inserted to a dropped position in the subject patient list.

Fig. 5D illustrates the screen displayed by the conference facilitating unit in the conference client apparatus 1 when the conference has started. If the user presses the conference start button 505 on the screen illustrated in Fig. 5A, the conference is started. Referring to Fig. 5D, a conference status 801 (i.e., "in conference" in the example of Fig. 5D) is displayed on the screen. A row 802 indicates the patient selected on the subject patient list display area 502. The patient to be the first subject is selected immediately after the conference is started. The selected patient is surrounded by a highlighted frame, and an open button is also highlighted. If the user presses a conference suspend button 803, the screen jumps to the screen illustrated in Fig. 13B to be described below. If the user presses a conference end button 804, the conference enters an end state.

Fig. 5E illustrates a screen in which the subject order has been changed, displayed by the conference facilitating unit in the conference client apparatus 1. Referring to Fig. 5E, the user drags and drops the patient 802 selected within the subject patient list display area 502. The user thus changes the order of the subjects to be discussed on in the conference. A row 901 indicates the selected patient after the order has been changed.

Fig. 5F illustrates a screen displayed by the conference facilitating unit in the conference client apparatus 1 before starting the conference on the subject number 4. More specifically, the screen illustrated in Fig. 5F is the screen displayed when the discussions on the subjects 1, 2, and 3 have ended. Referring to Fig. 5F, areas 1001, 1002, and 1003 respectively indicate the conclusion of the discussion on the subjects 1, 2, and 3. According to the present exemplary embodiment, the conclusions respectively indicated in the areas 1001, 1002, 1003 are "authorized", "authorized", and "additional information necessary". The conclusions are displayed to be easily understandable so that it is unnecessary to open the contents of the individual subjects. A summary generation button is displayed in an enabled state to the right of each of the areas 1001, 1002, and 1003 indicating the conclusions. The summary generation button is displayed in a disabled state in the case of a pending subject (i.e., the box indicating the conclusion is empty).

A row 1004 indicating the next patient to be discussed is surrounded by the highlighted frame, and the open button is also highlighted. If the user then presses the open button, the screen jumps to the screen illustrated in Fig. 6A to be described below.

Fig. 5G illustrates a different display screen of the patient list displayed by the conference facilitating unit in the conference client apparatus 1. The conference client apparatus 1 obtains the necessary information from the conference server 2. The conference client apparatus 1 then automatically displays the latest conference in which the user (i.e., the physician or the medical staff member) using the conference client apparatus 1 is set as a participant. This is similar to the screen illustrated in Fig. 5A.

Referring to Fig. 5G, material preparation status icons 3301 are similar to the material preparation status icons 506 illustrated in Fig. 5A. The material preparation status icons 3301 display the material preparation status with respect to each of the subject patients included in the subject patient list display area. In the display screen illustrated in Fig. 5G, the material preparation status icons 3304 indicate, from the left, the preparation statuses of the medical record information (i.e., the diagnosis and observations and the treatment plan), the examination image information (i.e., the DICOM image), the report information (i.e., the radiologic interpretation report or the pathological diagnosis report), and the camera image or the scheme figure. If the materials for the conference have not yet been prepared (e.g., the materials are not stored in the file server 3 or the data storage area 107), the material preparation status icons 3301 may be displayed differently according to whether the materials are ready, such as in a grayout state. The operator can thus easily recognize the lacking material.

A material adding button 3302 is for adding the materials from a clipboard. More specifically, the clipboard function is a general framework for receiving and transmitting the data between the applications. The clipboard function is used to retrieve the data which the image management system or other application has stored in the clipboard, as the conference material of the subject patient.

A material collection trigger button 3303 is a trigger button for collecting the materials. A material extraction rule (i.e., the condition expression) set for each conference is used to set the rule (the condition expression) for extracting the materials which are necessary in the conference from the electronic medical records and PACS. In other words, according to the present exemplary embodiment, the medical conference support system associates a material extraction rule with each conference, and stores the information thereon in a storage unit such as the data storage area 207. The unit for storing the above-described association information may be a storage unit other than the data storage area 207.

According to the present exemplary embodiment, the conditional expression defines at least one of the conditions such as the medical department of the image to be extracted, a period in which the image to be obtained has been captured, and the patient whose information is to be obtained. The conditional expression is not limited thereto, and other conditions may be employed. For example, a modality from which the information to be obtained has been acquired, or the physician from whom the information has been obtained may be added to the conditional expressions.

A specific example of the extraction condition will be described below.

### [The condition for extracting the materials for the respiratory unit preoperative conference]

For medical record articles, an article written within the past 30 days by an attending physician or the physician in charge, an admission summary, and an operation summary (if there is temporary registration) are extracted. Further, for the examination images, a chest X-ray among the 20 most-recently taken, an abdomen X-ray, computed tomography (CT), magnetic resonance imaging (MRI), and electrocardiogram are extracted. For laboratory test results, all data obtained in the past half year is extracted. Further, the pathologic reports, the image (radiologic interpretation) reports, and echo reports obtained in the past half year are extracted as a report. The materials are extracted so that determination of operating the subject patient, the operation method, and an appropriateness of the timing are verified from the view points of other physicians participating in the conference in addition to the attending physician and the physician in charge, in the preoperative conference performed before the operation.

For example, the CPU 102 may extract the materials based on the conditional expression. Further, the conditional expression may be determined based on the conference selected using the conference list button 602 on the screen illustrated in Fig. 5B and the conditional expression associated with each conference. The CPU 102 may then extract the material based on the determined conditional expression. In other words, the CPU 102 corresponds to the obtaining unit configured to obtain the medical information using the conditional expression determined as follows. The conditional expression is determined based on the conference selected by the selection unit and the information indicating the search condition associated with each of the plurality of conferences.

Further, such a material extraction rule is normally set so that the extraction is regularly performed as a batch process at night time when a load on the system in the medical organization is low. The date and time the material execution rule is executed is thus regulated. For example, if a medical department regularly holds the conference on Wednesday, the materials are extracted on Tuesday morning using the material execution rule. The operator may arbitrarily set the date and time when the material extraction rule is to be executed. Further, if the date and time of the conference has been input, the date and time when the material extraction rule is executed may be automatically set by preceding a predetermined number of days from the input date and time of the conference. The date and time of executing the material extraction rule can thus be easily set by the user only inputting the date and time of the conference, so that time and effort required for specifying the setting can be reduced.

If the material extraction rule is set to the conference and the user has operated the material collection trigger button 3303, the material extraction rule is applied to the patient. The group of conference materials based on the material extraction rule is then explicitly collected and updated. As a result, if a conference material has been newly added after the material extraction rule has been last applied (normally at night time), the newly added conference material can be extracted and added to the group of conference materials. The user may extract the material by pressing the material collection trigger button 3303 after the materials have been regularly extracted. In such a case, the materials other than the previously extracted materials may be collected, or all of the materials may be re-extracted and written over the previously extracted materials.

Further, if a new material has been added to the server, e.g., the PACS server 4, after the material extraction has been regularly performed and before starting the conference, the material collection trigger button 3303 may be highlighted to indicate that there is a newly added material. The material collection trigger button 3303 may be highlighted by blinking or by displaying using a different color. The operator can thus easily recognize whether there is a new material in the server. Furthermore, when the information, e.g., an image, which is considered important is to be input to the server, e.g., the PACS server 4, a flag may be set to the information. The material collection trigger button 3303 may then be highlighted only in the case where the information the flag is set thereto is input to the server after the regular material extraction has been performed and before starting the conference. As a result, all of the important images can be used in the conference, and the patient can be more appropriately treated. Since the conference client apparatus 1 is connected to the various servers via the network 9, whether the flag is set to the information can be determined.

When the user presses a patient list update button 3304, the conference facilitating unit obtains the patient list of the conference from the conference server and displays it on the screen.

If the user presses a subject display button 3305 which is similar to the subject display button 507 on the screen illustrated in Fig. 5A, the screen jumps to the screen displaying the detail information on the subject patient illustrated in Fig. 6A to be described below.

Fig. 6A illustrates a screen displayed by the conference facilitating unit in the conference client apparatus 1 when the discussion on the subject number 4 has been started. If the user presses the open button 805 on the screen illustrated in Fig. 5D, the detailed information on the subject patient is displayed. The physician in charge refers to the displayed detailed information. The physician can then sequentially confirm and describe the patient basic information, the diagnosis information (the diagnosis and observations in the medical record information and the treatment plan), and the examination data which is the basis of the diagnosis and observations and the treatment plan.

The order of the subjects in the conference and the conclusion of the discussion on each subject patient are indicated in the upper portion of a subject state indicator 1101. According to the present exemplary embodiment, the subject is the fourth subject among the 10 subjects in the entire conference. The discussions on the previous 3 cases have been resolved, and the remaining 6 cases are pending. If the user inputs the conclusion in a conclusion input area 1107, the case becomes resolved. As a result, if there is an emergency call during the conference and the physician in charge leaves the conference, the conference may proceed without inputting the conclusion on the patient. In such a case, the screen may indicate that the conference is proceeding while there is a pending subject as on the screen illustrated in Fig. 6C. The names of the patient to be the next subject and the physician in charge are indicated in the lower portion of the subject state indicator 1101. Patient basic information 1102 displays the patient ID and name, sex, age, and the physician in charge, similarly as in the subject patient area 502 on the screen illustrated in Fig. 5A. A diagnosis information display area 1103 displays subject object assessment plan (SOAP) information extracted from the electronic medical record.

A conference material display area 1104 displays as a list the conference materials prepared by the conference preparation unit in the conference client apparatus 1. Each conference material is one of the examination image information, the report information, or other information, and the conference materials are listed for each type. A thumbnail image, an examination date, a person in charge of preparing the material, and an external system call button are displayed on each material in the list. If the user presses the external system call button, the external system such as the electronic medical records or the PACS viewer is activated, and the original material of the conference material can be referred to.

A work area 1105 displays the conference material selected by the user dragging and dropping the conference material from the conference material display area 1104. Conference material display options in the work area are arranged in the lower portion of the work area 1105. More specifically, a single material display switching button, a two materials comparison display switching button, an annotation pen selection button, an annotation pen color change button, and an eraser button are arranged from the left (to be described below with reference to Figs. 9A, 9B, and 9C).

A summary materials display area 1106 displays as a list the thumbnails of the conference materials to be included in the summary. The conference materials in the summary materials display area 1106 are stored along with the annotations, and transmitted to the summary generation unit. The conclusion input area 1107 is used for easily inputting the conclusion of the discussion by the physicians with respect to the diagnosis and observations and the treatment plan, and the examination data which is the basis thereof. According to the present exemplary embodiment, the user selects any one of "authorized", "additional information necessary", and "continue".

Material switching buttons 1108 are used for selecting and switching any one of the examination image information, the report information, and other information, the type of the material to be displayed on the conference material display area 1104. A conference material display toggle button 1109 is used for switching between displaying/not displaying the conference material display area 1104 (as illustrated in Fig. 10E to be described below). A diagnosis information display toggle button 1110 is used for switching between displaying/not displaying the diagnosis information display area 1103 (as illustrated in Fig. 10B to be described below). A conference notes display toggle button 1111 is used for switching between displaying/not displaying the area for inputting the conference notes on the patient.

An update reflecting button 1112 reflects on the screen the information changed by an operation other than displaying the screen (e.g., a background job).

An electronic medical record activation button 1113 disposed in the diagnosis information display area 1103 is used for activating the electronic medical record and referring to the original information on the patient in the electronic medical record. A material addition button 1114 disposed in the conference material display area 1104 is used for activating a file selection dialog for adding the lacking material. A summary generation button 1115 is used for calling the conference summary output unit and generating the summary of the discussions on the patient. A close button 1116 is used for closing the screen on the detailed information on the subject patient, so that the screen jumps to the subject list display screen illustrated in Fig. 5A.

Fig. 6B illustrates a screen displayed by the conference facilitating unit in the conference client apparatus 1 in which the material list (i.e., an examination image tab) has been scrolled. Referring to Fig. 6B, if the user performs a vertical drag operation on a conference material display area 1201, the items on the list are scrolled in the vertical direction. Further, if the user performs a vertical flicking operation on the conference material display area 1201, the items on the list are inertial-scrolled in the vertical direction.

Fig. 6C illustrates another example of the screen on the patient details displayed by the conference facilitating unit in the conference client apparatus 1. Referring to Fig. 6C, the conference facilitating unit displays the detailed information on the subject patient. The physician in charge refers to the displayed detailed information. The physician can then sequentially confirm and describe the patient basic information, the diagnosis information (the diagnosis and observations and the treatment plan in the medical record information), and the examination data which is the basis of the diagnosis and observations and the treatment plan.

External system call buttons 3401 for each patient are used for activating the electronic medical records and the PACS viewer, and displaying a top page, for example, with respect to the patient in each system. According to the display screen example illustrated in Fig. 6C, the following external system call buttons 3401 are arranged from the left: the buttons for the medical record information (i.e., the diagnosis and observations and the treatment plan), the examination image information (i.e., the DICOM image), the report information (i.e., a radiologic interpretation report or a pathological diagnosis report), and for other information including the camera image and the scheme figure.

An external system call button 3402 for each conference material is used for activating the electronic medical records and the PACS viewer according to the type of each conference material in the material list, and referring to the original conference material. In other words, if the user selects one of the external system call button 3401 for each patient, the top page with respect to the patient who is the subject of the conference is opened. On the other hand, if the user selects the external system call button 3402 for each conference material, the conference material such as the image is directly opened. As described above, since the buttons are disposed for opening different layers when the external system is called, the operator can more flexibly perform the operations, and user-friendliness is thus improved. Further, since the buttons for opening the different layers are disposed, the time necessary for searching for the desired image is shortened as compared to when only one of the buttons is disposed.

A material adding button 3403 for adding the material from the clip board operates similarly as the material adding button 3302 illustrated in Fig. 5G.

A material collection trigger button 3404 is used similarly as the material collection trigger button 3303 illustrated in Fig. 5G.

The order of the subject in the conference and the conclusion of the discussion on the subject patient are indicated in the upper portion of a subject status indicator 3405. According to the present exemplary embodiment, the subject is fifth subject among the 8 subjects in the entire conference. The discussions on the previous 3 cases have been resolved, and the fourth case, the sixth case, and the cases thereafter are pending. When the user selects and inputs from a pull-down menu the conclusion in a conclusion input area 3406, the case becomes resolved. For example, if there is an emergency call during the conference and the physician in charge of the fourth patient leaves the conference, the conference may proceed without inputting the conclusion on the patient. If the discussion has then been resolved, the subject status indicator 3405 is checked. Further, it is assumed that the first patient in the patient list illustrated in Fig. 5G is selected, and the screen jumps to the display screen illustrated in Fig. 6C. If the user then resolves the discussion using the conclusion input area 3406, the screen returns to the display screen illustrated in Fig. 5G. In such a case, the second patient in the patient list illustrated in Fig. 5G is in a selected state. In other words, when the discussion is resolved on the screen illustrated in Fig. 6C, the next patient is selected on the screen illustrated in Fig. 5G. On the other hand, if the discussion has not been resolved and the user selects the "close" button displayed on the left side of the conclusion input area 3406, the screen jumps to the display screen illustrated in Fig. 5G. However, in such a case, the second patient is not selected, and the first patient remains selected. Further, the subject status indicator 3405 is not checked. As a result, when the screen jumps to the screen illustrated in Fig. 5G, the patient to be selected is different according to the operation which has triggered the screen to jump thereto.

As described above, if the discussion has not been resolved, the next patient is not to be in a selected state. It thus prevents the conference on the next subject to be held without resolving the previous conference, and the conference with respect to the first patient from not becoming concluded.

Fig. 7A illustrates a selected material displayed in a workspace on the screen displayed by the conference facilitating unit in the conference client apparatus 1. Referring to Fig. 7A, the screen displays a material 1301 which has been selected in the conference material display area. A highlighted frame is displayed on the thumbnail portion of the selected material. The selected material is displayed on the workspace as a material 1302. The method for displaying the selected material in the workspace will be described below with reference to Fig. 7B. The workspace displays the reference image (including a greater data amount as compared to the thumbnail image) of the selected material. An annotation all-erasing button 1303 is used for deleting all of the annotation information added to the material. This operation is different from the eraser button in the lower portion of the work area 1105 illustrated in Fig. 6A which only erases the selected annotation.

The user designates whether to allow the material to be reflected in the summary by using a summary storing check box 1304. The designated material is displayed in the summary material display area 1106 when the material is closed. An external reference button 1305 is for activating the external system (i.e., the electronic medical records or the PACS viewer) for referring to the original material. A close button 1306 for closing the material displayed in the workspace closes and deletes the designated material from the work area.

Fig. 7B illustrates a drag-and-drop operation of a material from the material list on the screen displayed by the conference facilitating unit in the conference client apparatus 1. Referring to Fig. 7B, a thumbnail display 1401 is used for operating the material selected in the conference material display area. The user drags and drops the selected material, and the material is displayed in the workspace. A "+" icon indicating that the thumbnail image is being operated is superimposed and displayed on the thumbnail display 1401 which is translucently displayed.

Fig. 8A illustrates a material list (i.e., the report tab) displayed on the screen displayed by the conference facilitating unit in the conference client apparatus 1. Referring to Fig. 8A, if the user presses a report tab 1501 among the material switching buttons, the following operation is performed. The materials classified as the report (i.e., the examination report) among the materials prepared as the conference materials are displayed as a list in the conference material display area. According to the present exemplary embodiment, the system handles the reports similarly as the examination images. The materials in the conference material display area are similarly displayed in the workspace by the drag-and-drop operation.

Fig. 8B illustrates a material list (i.e., a non-DICOM image tab) displayed on the screen displayed by the conference facilitating unit in the conference client apparatus 1. Referring to Fig. 8B, if the user presses a non-DICOM image tab 1601 among the materials switching buttons, the following operation is performed. The materials classified as the non-DICOM image (i.e., the camera images or personal computer (PC) images) and the document (i.e., texts or various office documents) among the materials prepared as the conference materials are displayed as a list in the conference material display area. According to the present exemplary embodiment, the system handles the non-DICOM images and the documents similarly as the examination images. The materials in the conference material display area are similarly displayed in the workspace by the drag-and-drop operation.

Fig. 9A illustrates an annotation added to a material and an automatic summary setting on the screen displayed by the conference facilitating unit in the conference client apparatus 1. Referring to Fig. 9A, an annotation designation button 1701 is used for switching between an annotation input mode and a pointing mode in the workspace by a toggle operation. An example of an annotation input result 1702 indicates that the input result of a stroke which has been input using the mouse or a touch device is superimposed and displayed on the examination image data. A summary store check box 1703 is used for storing the set materials for generating the summary in the summary material display area 1106. The check box of the annotated material is automatically set, so that the burden on the user is reduced. On the other hand, if the user has explicitly cleared the check box which has been automatically set, the annotated material is not stored in the summary material display area 1106. An eraser button 1704 is used for deleting the designated annotation portion. An annotation all-erasure button 1705 is used for deleting all of the annotation information.

Fig. 9B illustrates a screen for performing annotation color selection displayed by the conference facilitating unit in the conference client apparatus 1.

Referring to Fig. 9B, a color selection button 1801 is used for designating an input color of the annotation employing a color selection panel 1802. As a result, the annotations in different colors can be set, and a discussion process in the conference can be expressed to be easily understandable (as illustrated in Fig. 9C).

Fig. 9D illustrates a scaled display of the subject materials on the screen displayed by the conference facilitating unit in the conference client apparatus 1. More specifically, Fig. 9D illustrates an example of an enlarged conference material 2001 obtained by enlarging an annotated area in the conference material illustrated in Fig. 9C by the user operation on the workspace. If a pinch-in/pinch-out operation (i.e., a pinching operation using two fingers) or a mouse wheel operation by the user is detected in a designated area, the conference material is enlarged or reduced. Further, the display area is moved by the user operation on the designated area in the workspace (not illustrated). According to the present exemplary embodiment, the display area is moved by a drag operation.

Fig. 10A illustrates the materials set to be used in the summary on the screen displayed by the conference facilitating unit in the conference client apparatus 1.

Referring to Fig. 10A, a list including conference materials 2101, 2102, 2103 is a list of conference materials to be reflected in the summary stored in the summary material display area 1106. The progress status of the conference becomes visible by displaying the materials, as a list, which has been annotated or explicitly designated to be used in the summary during the conference. According to the present exemplary embodiment, the result of the operation performed on the workspace during the conference, such as annotating, enlarging, or reducing the material, or shifting the display area is displayed as the thumbnail. The result of the operation performed during the conference is reflected in the summary similarly as in the thumbnail.

Fig. 10B illustrates a case where the diagnosis information display area is not displayed on the screen displayed by the conference facilitating unit in the conference client apparatus 1. Referring to Fig. 10B, the user operates the diagnosis information display toggle button 1110 on the screen illustrated in Fig. 6A, so that the diagnosis information display area 1103 is in non-displayed state on the screen. In such a case, the summary material display area 1106 is horizontally shifted to the left, so that the workspace is enlarged. As a result, the conference material can be displayed in a larger space and discussed on, so that the conference becomes easier to understand.

Fig. 10C illustrates an example of dividing a screen into 2 screens for displaying the materials on the screen displayed by the conference facilitating unit in the conference client apparatus 1. Referring to Fig. 10C, if the user presses a two-screen division button 2301, the workspace area is divided into two screens. Each of the divided workspaces can then display the conference material, and the user can add annotations to or enlarge or reduce the images thereon (refer to Fig. 10D). The materials can thus be easily compared. In contrast, a single screen button 2302 causes the divided screens to become one screen. According to the present exemplary embodiment, a left screen is caused to remain, and a right screen is deleted.

Fig. 10E illustrates a case where a material list is not displayed with respect to the two-screen display of the materials on the screen displayed by the conference facilitating unit in the conference client apparatus 1. Referring to Fig. 10E, the user operates the conference material display toggle button 1109, so that the conference material display area 1104 becomes in a non-displayed state on the screen. In such a case, the workspace is enlarged. The conference material can thus be displayed in a larger space and discussed on, so that the conference becomes easier to understand. The diagnosis information display area 1103 is also not displayed on the screen illustrated in Fig. 10E. However, the conference material display toggle button 1109 and the diagnosis information display toggle button 1110 can be operated independent of each other. In other words, the diagnosis information display area 1103 can be displayed while the conference material display area 1104 is not displayed.

Fig. 11 illustrates a non-DICOM image displayed on the screen displayed by the conference facilitating unit in the conference client apparatus 1. When the discussion is being held using the non-DICOM image, the non-DICOM image can be dragged and dropped in the workspace by the operation similar to the operation performed with respect to the examination image. The user can thus add the annotations to and enlarge or reduce the image, move the display area, and divide the screen (to compare the materials) .

Fig. 12A illustrates an easy input of a conclusion on the screen displayed by the conference facilitating unit in the conference client apparatus 1. As described above, the user inputs, using the conclusion input area 1107, the conclusion obtained when the physicians have held the discussion with respect to the diagnosis and observations and the treatment plan, and the examination data which is the basis thereof. As a result, if an agreement has been reached, the user can simply select "authorized" (as illustrated in Fig. 12B) and input the conclusion. Similarly, if it is necessary to separately add a comment, the user selects "necessary to add information", and if it is necessary to continue investigations, the user selects "continue". The conference summary output unit then reflects the input conclusion in the summary.

Fig. 12C illustrates adding a comment on the screen displayed by the conference facilitating unit in the conference client apparatus 1. The user operates the conference note display toggle button 1111 and switches between displaying/not displaying a conference note input area with respect to the patient. Referring to Fig. 12C, the user can input text information in a conference note input area 2901. The user can easily designate in a remark type selection box 2902 the type of the remark, e.g., a question, an answer, an action item, a comment, or a conclusion. A speaker selection box 2903 can be used to easily designate the speaker from the conference participants. A conference note area 2904 displays the contents of each of the input remarks as a list. The notes on the remarks recorded in the conference note area 2904 are transferred to the summary generation unit along with the materials referred to in the conference which are stored in the summary material display area 1106.

Fig. 13A illustrates a screen displayed by the conference facilitating unit in the conference client apparatus 1 after the conference on the subject has been held and in which the next subject is highlighted. After the discussions using the screen displaying the subject number 4 as described above, the user inputs the conclusion from the conclusion input area 1107 and operates the close button 1116 on the screen illustrated in Fig. 6A. The system then returns from displaying the screen on the individual subject to displaying the subject patient list as illustrated in Fig. 13A. In such a case, the system determines that the discussion on the subject number 4 has ended, and automatically selects the subject number 5 as the next subject. The system highlights the subject number 5 to prompt start of the conference on the subject, and focuses on the open button 805.

Fig. 13B illustrates a screen displayed by the conference facilitating unit in the conference client apparatus 1 when the conference has been suspended. If the user presses the conference suspend button 803 on the screen while the subject patient list is being displayed, the screen indicates that the conference is suspended as illustrated in Fig. 13B. Referring to Fig. 13B, a conference restart button 3101 is displayed when the conference has been suspended. If the user then presses the conference restart button 3101, the conference is restarted (as illustrated in Fig. 13C).

According to the present exemplary embodiment, the above-described configurations enables the medical conference support system to facilitate the conference by collaborating with the electronic medical records, PACS, the examination report system, the image management system, and the file server in the hospital. In particular, according to the present exemplary embodiment, the explicit material preparation trigger employing the material collection button, the relation between the conclusion input and the conference progress status display, and reference to the external system are the features thereof.

According to the present exemplary embodiment, the operations for enlarging or reducing and moving the display area are performed on the workspace which has been divided into two screens, the divided workspaces are processed independent of each other. However, the process performed with respect to the workspaces is not limited thereto. For example, one of the divided workspace may be enlarged or reduced, or the display area thereof may be moved in conjunction with the other divided workspace.

Further, according to the present exemplary embodiment, when the discussion employing the detail screen of one patient ends, the screen once returns to the patient list display screen, and the next patient is selected. However, a screen transition method is not limited thereto. For example, the screen may directly jump from the detail screen of one patient to the detail screen of the next patient.

Furthermore, according to the present exemplary embodiment, filtering and sorting processes performed with respect to the material list displayed in the subject patient detail screen are not described. However, the displayed material list may be filtered and sorted. Moreover, according to the present exemplary embodiment, the respiratory unit operation conference held before the operation has been described as an example. However, the types of conferences are not limited thereto. For example, if the present invention is to be applied to a cardiovascular unit conference held before the patient is to be discharged from the hospital (held from 8:30 to 9:30 every Friday), the following patient extraction condition and the material extraction condition may be employed.
[The condition for automatically extracting a patient to be the subject of the cardiovascular unit conference before discharge]
The patient is to be discharged from the department or is to be transferred from the department to other department during the period of one week from the date of the conference is extracted as the subject patient. The patient is extracted as described above for discussing recovery of the patient and whether home care is allowable, by considering the opinions of other physicians participating in the conference.

### [The condition for extracting the materials for the cardiovascular unit conference before discharge]

For medical record articles, the article written by the attending physician or the physician in charge of the department after admission, the admission summary, the operation summary, a discharge summary (if there is temporary registration), and a department transfer summary (if there is temporary registration) are extracted. Further, for the examination images, the chest X-ray, the abdomen X-ray, CT, MRI, and electrocardiogram taken at admission, before and after the operation, and most recently are extracted. For laboratory test results, all data obtained since admission is extracted. Further, the pathologic reports, the image (radiologic interpretation) reports, and the echo reports obtained since admission are extracted. The materials are extracted so that determination of discharging the subject patient is verified based on the symptoms of the subject patient at admission and the recovery after treatment in the pre-discharge conference held before discharging the patient, from the view points of other physicians participating in the conference in addition to the attending physician and the physician in charge.

### Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™) , a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A medical conference support system (1,2) comprising:
a selection means configured to select a conference from a plurality of conferences; and
an obtaining means configured to obtain medical information (302) using a search condition determined based on a conference selected by the selection means and information indicating a search condition associated with each of the plurality of conferences.

2. The conference support system according to claim 1, wherein the obtaining means is operable to obtain link information for linking to original information of an external apparatus from which the medical information is to be obtained.

3. The conference support system according to claim 1 or 2,
wherein the search condition is a date and time at which the selected conference is regularly held, and
wherein the obtaining means is operable regularly to obtain the medical information at date and time determined based on the date and time the selected conference is regularly held.

4. The conference support system according to any one of claims 1 to 3, further comprising an instruction means for receiving an instruction from a user,
wherein the obtaining means is operable to obtain the medical information according to the instruction.

5. The conference support system according any one of claims 1 to 4, further comprising a display control means for causing a display means to display the medical information obtained by the obtaining means.

6. The conference support system according to claim 5, wherein the obtaining means is further operable to obtain a subject patient list and conference materials for the selected conference, and
wherein the display control means is operable to switch a display state of the subject patient list according to the conference materials obtained by the obtaining means.

7. A conference support method for supporting a medical conference comprising:
selecting a conference from a plurality of conferences; and
obtaining medical information using a search condition determined based on the selected conference and information indicating a search condition associated with each of the plurality of conferences.

8. The conference support method according to claim 7, further comprising obtaining link information for linking to original information of an external apparatus from which the medical information is to be obtained.

9. The conference support method according to claim 7 or 8, wherein the search condition is a date and time at which the selected conference is regularly held, and
further comprising regularly obtaining the medical information at date and time determined based on the date and time the selected conference is regularly held.

10. The conference support method according to any one of claims 7 to 9, further comprising:
receiving an instruction from a user; and
obtaining the medical information according to the instruction.

11. The conference support method according to any one of claims 7 to 10, further comprising performing display control for causing a display means to display the obtained medical information.

12. The conference support method according to any one of claims 11, further comprising:
further obtaining a subject patient list and conference materials for the selected conference; and
switching, when performing display control, a display state of a subject patient list according to the obtained conference materials.

13. A computer executable program for causing a computer to execute the conference support method according to any one of claims 7 to 12.
